# EUROPEAN PATENT APPLICATION

(11) **EP 4 682 150 A1**
(43) Date of publication of application: **21.01.2026**
(21) Application number: 25189733.6
(22) Date of filing: 15.07.2025
(51) Int. Cl.: C07D 493/04, H10K 50/00

(54) **HETEROCYCLIC COMPOUND AND ORGANIC LIGHT-EMITTING DEVICE INCLUDING THE SAME**

(30) Priority: 17.07.2024 KR 20240094507
(71) Applicant: LT Materials Co., Ltd., Yongin-si Gyeonggi-do 17118 (KR)
(72) Inventor: SEO, Jin Gwan, 17118 Yongin-si (KR); JEONG, Won Jang, 17118 Yongin-si (KR); KIM, Dong Jun, 17118 Yongin-si (KR); CHOI, Dae Hyuk, 17118 Yongin-si (KR)
(74) Representative: Meissner Bolte Partnerschaft mbB

(57) **Abstract**

According to embodiments of the present invention, a heterocyclic compound represented by specific chemical formulas is provided. The organic light-emitting device includes a first electrode, an organic layer, and a second electrode, and the organic layer includes the above-described heterocyclic compound.

## Description

### CROSS-REFERENCE TO RELATED APPLICATION AND CLAIM OF PRIORITY

This application claims priority to Korean Patent Application No. 10-2024-0094507 filed on July 17, 2024 in the Korean Intellectual Property Office (KIPO), the entire disclosure of which is incorporated by reference herein.

### [BACKGROUND OF THE INVENTION]

### 1. Field of the Invention

The present disclosure relates to a heterocyclic compound and an organic light-emitting device including the same.

### 2. Description of the Related Art

An organic light-emitting display (OLED) device includes organic light-emitting devices having self-emissive characteristics. The organic light-emitting display device does not require a separate light source, and may provide a wide viewing angle and fast response speed, while also improving contrast and brightness.

In the organic light-emitting device, an organic light-emitting layer is formed for each pixel, and the organic light-emitting layer may be interposed between electrodes facing each other. Holes and electrons injected from each electrode recombine in the organic light-emitting layer to generate excitons, and light may be generated through the energy released from the excitons.

In order to implement an organic light-emitting device with high efficiency and long lifetime, materials applied to the organic light-emitting layer have been studied.

### [SUMMARY OF THE INVENTION]

An object of the present disclosure is to provide a novel heterocyclic compound.

Another object of the present disclosure is to provide an organic light-emitting device including the heterocyclic compound.

A heterocyclic compound according to the present disclosure is represented by Formula 1 below: 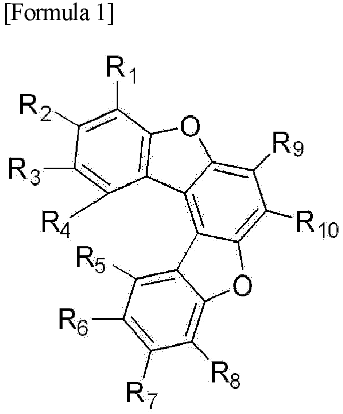 in Formula 1, one of R₁ to R₈ is a substituent represented by Formula 2 below, and
the others are each independently selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -SiRR'R"; and -P(=O)RR',
R, R' and R" are each independently hydrogen; deuterium; -CN; a substituted or unsubstituted alkyl group having 1 to 60 carbon atoms; a substituted or unsubstituted cycloalkyl group having 3 to 60 carbon atoms; a substituted or unsubstituted aryl group having 6 to 60 carbon atoms; or a substituted or unsubstituted heteroaryl group having 2 to 60 carbon atoms, 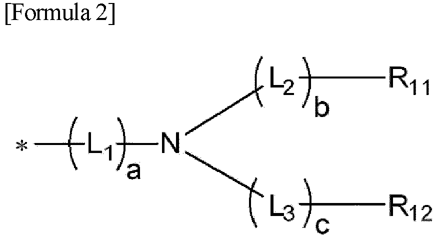
in Formula 2, R₁₁ and R₁₂ are each independently a substituted or unsubstituted aryl group having 6 to 60 carbon atoms, or a substituted or unsubstituted heteroaryl group having 2 to 60 carbon atoms,
L₁, L₂ and L₃ are each independently a direct linkage, a substituted or unsubstituted arylene group having 6 to 60 carbon atoms, or a substituted or unsubstituted heteroarylene group having 2 to 60 carbon atoms,
a, b and c are each independently 0 or an integer from 1 to 5,
when a is an integer from 2 to 5, a plurality of L₁ are the same or different,
when b is an integer from 2 to 5, a plurality of L₂ are the same or different,
when c is an integer from 2 to 5, a plurality of L₃ are the same or different,
one of R₉ and R₁₀ is -(L₄)n-R₁₃, and the other is hydrogen or deuterium,
L₄ is a direct linkage, a substituted or unsubstituted arylene group having 6 to 60 carbon atoms, or a substituted or unsubstituted heteroarylene group having 2 to 60 carbon atoms,
n is an integer from 1 to 5, and when n is an integer from 2 to 5, a plurality of L₄ are the same or different,
R₁₃ is a substituted or unsubstituted aryl group having 6 to 60 carbon atoms, or a substituted or unsubstituted heteroaryl group having 2 to 60 carbon atoms, and
* indicates a bonding site with an adjacent atom.

An organic light-emitting device according to the present disclosure includes: a first electrode; a second electrode disposed on the first electrode; and one or more organic layers interposed between the first electrode and the second electrode. One or more of the organic layers include the heterocyclic compound.

The heterocyclic compound according to the present disclosure may be used as an organic layer material for an organic light-emitting device. The heterocyclic compound may have a 5-membered fused ring structure including a benzofuran moiety, and may have appropriate hole or electron mobility by including an aryl group bonded to the third ring and an aminoaryl group bonded to the terminal ring. Accordingly, the stability of the compound may be improved, and the compound may resist decomposition or transformation during operation of the organic light-emitting device, thereby enabling efficient transfer of electrons or holes.

In addition, the band gap may be controlled by adjusting the bonding site of the substituents of the heterocyclic compound, and it may be suitably used as a material for a hole transport layer, an auxiliary hole transport layer, or an electron blocking layer of an organic light-emitting device.

The organic light-emitting device including the heterocyclic compound may have a low operating voltage, high luminous efficiency, and/or improved lifetime characteristics.

### [BRIEF DESCRIPTION OF THE DRAWINGS]

The above and other objects, features and other advantages of the present disclosure will be more clearly understood from the following detailed description taken in conjunction with the accompanying drawings, in which:

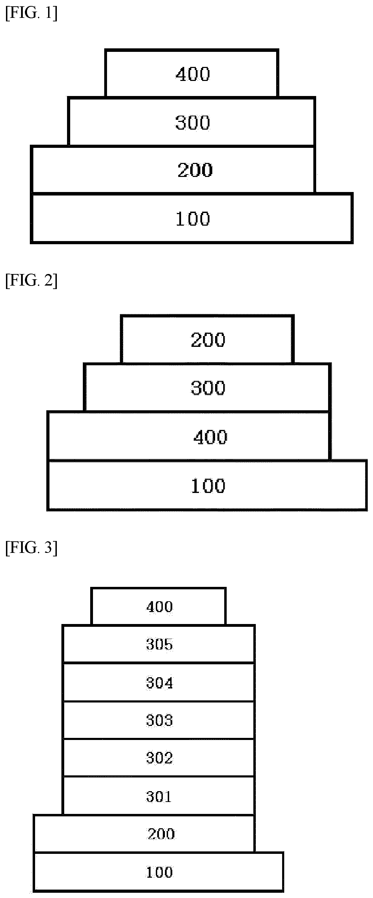

FIGS. 1 to 3 are schematic views illustrating a stacked structure of an organic light-emitting device according to an embodiment of the present disclosure, respectively.

### [DETAILED DESCRIPTION OF THE INVENTION]

Hereinafter, embodiments of the present disclosure will be described in detail with reference to the accompanying drawings. However, these embodiments are merely examples, and the present disclosure is not limited to the specific embodiments described as examples.

In this specification, when a part is said to "include" or "comprise" a component, this refers to the fact that the part may further include other components, unless specifically stated otherwise, and does not exclude the presence of additional components.

In this specification, the * symbol in a chemical formula indicates a bonding site.

In this specification, "substituted" refers to a case where any hydrogen atom bonded to a carbon atom of a compound is replaced with another substituent. The position to be substituted is not limited as long as it is a position where a hydrogen atom may be substituted, that is, a position where a substituent may be introduced. When two or more substitutions occur, the substituents may be the same or different.

In this specification, "substituted or unsubstituted" refers to substitution with, or the absence of substitution by, one or more substituents selected from the group consisting of deuterium; a halogen group; -CN; an alkyl group having 1 to 60 carbon atoms; an alkenyl group having 2 to 60 carbon atoms; an alkynyl group having 2 to 60 carbon atoms; a haloalkyl group having 1 to 60 carbon atoms; an alkoxy group having 1 to 60 carbon atoms; an aryloxy group having 6 to 60 carbon atoms; an alkylthioxy group having 1 to 60 carbon atoms; an arylthioxy group having 6 to 60 carbon atoms; an alkylsulfoxy group having 1 to 60 carbon atoms; an arylsulfoxy group having 6 to 60 carbon atoms; a cycloalkyl group having 3 to 60 carbon atoms; a heterocycloalkyl group having 2 to 60 carbon atoms; an aryl group having 6 to 60 carbon atoms; a heteroaryl group having 2 to 60 carbon atoms; one or more substituents selected from the group consisting of -SiRR'R"; -P(=O)RR'; and -NRR', or a substituent in which two or more of substituents selected from the above-exemplified substituents are linked. R, R' and R" are each independently a substituent consisting of at least one of hydrogen; deuterium; a halogen group; an alkyl group; an alkenyl group; an alkoxy group; a cycloalkyl group; a heterocycloalkyl group; an aryl group; and a heteroaryl group.

In this specification, if a substituent is not indicated in a chemical formula or compound structure, it refers to that a hydrogen atom is bonded to the corresponding carbon atom. However, since deuterium (²H, D) is an isotope of hydrogen, some hydrogen atoms may be deuterium atoms.

In this specification, if a substituent is not indicated in a chemical formula or compound structure, it may mean that all possible substituent positions are occupied by hydrogen or deuterium. That is, since deuterium is an isotope of hydrogen, some of the hydrogen atoms may be the isotope deuterium, and in this case, the deuterium content may range from 0% to 100%.

If no substituent is indicated in the chemical formula or compound structure, hydrogen and deuterium may coexist in the compound, unless deuterium is explicitly excluded, for example, when the deuterium content is 0%, the hydrogen content is 100%, or all substituents are hydrogen.

Deuterium is one of the isotopes of hydrogen, and is an element having a deuteron, consisting of one proton and one neutron, as its nucleus. It may be expressed as hydrogen-2, and its element symbol may be written as D or²H.

Isotopes are atoms with the same atomic number (Z) but different mass numbers (A). That is, isotopes can also be interpreted as elements having the same number of protons but differing in the number of neutrons.

In this specification, the meaning of T% content of a specific substituent may be defined as T2 / T1 × 100 = T(%) where T1 represents the total number of substituent positions that the base compound may have, and T2 represents the number of the specific substituent among them.

In one example, a deuterium content of 20% in a phenyl group, represented by 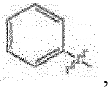 refers to that the total number of substitutable positions on the phenyl group is 5 (T1 in the formula), and the number of deuterium atoms is 1 (T2 in the formula), resulting in 20% content. That is, cases where the phenyl group has a deuterium content of 20% may be represented by the following structural formulas.

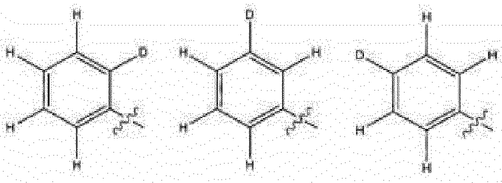

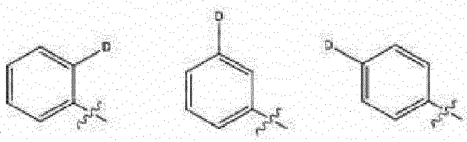

In addition, "a phenyl group having a deuterium content of 0%" may refer to a phenyl group having 5 hydrogen atoms and no deuterium atoms.

In this specification, the term "halogen" may refer to fluorine, chlorine, bromine or iodine.

In this specification, an alkyl group may include a straight-chain or branched-chain having 1 to 60 carbon atoms, and may be further substituted with another substituent. The number of carbon atoms in the alkyl group may be 1 to 60, 1 to 40, or 1 to 20. Examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethylpropyl group, an isohexyl group, a 2-methylpentyl group, a 4-methylhexyl group, and a 5-methylhexyl group, but are not limited thereto.

In this specification, an alkenyl group may include a straight-chain or branched-chain having 2 to 60 carbon atoms, and may be further substituted with another substituent. The number of carbon atoms in the alkenyl group may be 2 to 60, 2 to 40, or 2 to 20. Examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl group, a 2-phenylvinyl group, a 2,2-diphenylvinyl group, a 2-phenyl-2-(naphthyl)vinyl group, a 2,2-bis(diphenyl)vinyl group, a stilbenyl group, and a styrenyl group, but are not limited thereto.

In this specification, an alkynyl group may include a straight-chain or branched-chain having 2 to 60 carbon atoms, and may be further substituted with another substituent. The number of carbon atoms in the alkynyl group may be 2 to 60, 2 to 40, or 2 to 20.

In this specification, a haloalkyl group refers to an alkyl group substituted with a halogen group, and examples thereof include -CF₃, -CF₂CF₃, etc., but are not limited thereto.

In this specification, a cycloalkyl group may include a monocyclic or polycyclic group having 3 to 60 carbon atoms, and may be further substituted with another substituent. Here, a polycyclic group refers to a group in which a cycloalkyl group is directly connected to or fused with another ring group. In this case, the other ring group may be a cycloalkyl group, but may also be a different type of ring group, such as a heterocycloalkyl group, an aryl group, a heteroaryl group, etc. The number of carbon atoms in the cycloalkyl group may be 3 to 60, 3 to 40, or 5 to 20. Examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, etc., but are not limited thereto.

In this specification, a heterocycloalkyl group includes at least one heteroatom selected from O, S, Se, N and Si, and may include a monocyclic or polycyclic group having 2 to 60 carbon atoms, and may be further substituted with another substituent. Here, the polycyclic group refers to a group in which a heterocycloalkyl group is directly connected to or fused with another ring group. In this case, the other ring group may be a heterocycloalkyl group, but may also be a different type of ring group, for example, a cycloalkyl group, an aryl group, a heteroaryl group, etc. The number of carbon atoms in the heterocycloalkyl group may be 2 to 60, 2 to 40, or 3 to 20.

In this specification, an aryl group may include a monocyclic or polycyclic group having 6 to 60 carbon atoms, and may be further substituted with another substituent. Here, the polycyclic group refers to a group in which an aryl group is directly connected to or fused with another ring group. In this case, the other ring group may be an aryl group, but may also be a different type of ring group, such as a cycloalkyl group, a heterocycloalkyl group, a heteroaryl group, etc. The aryl group includes a spiro group structure. The number of carbon atoms in the aryl group may be 6 to 60, 6 to 40, or 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, and fused ring groups thereof, etc., but are not limited thereto.

In this specification, the terphenyl group may be selected from the following structures.

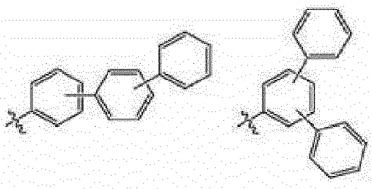

In this specification, the fluorenyl group may be substituted, and adjacent substituents may be bonded to each other to form a ring.

The substituted fluorenyl group may be represented by the following structural formula, but is not limited thereto.

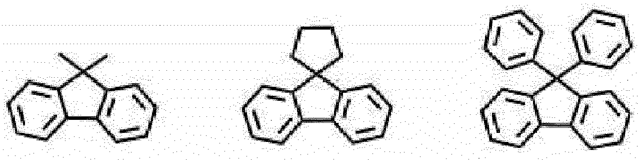

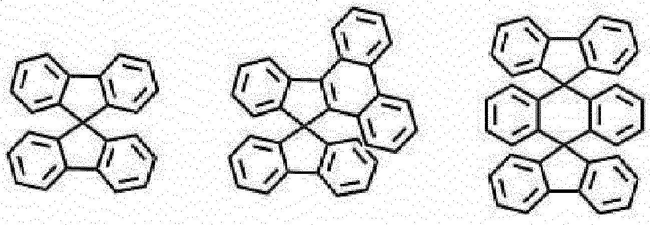

In this specification, an alkoxy group may be represented by -O(R101), and R101 may be selected from the examples of the alkyl group described above.

In this specification, an aryloxy group may be represented by -O(R102), and R102 may be selected from the examples of the aryl group described above.

In this specification, an alkylthioxy group may be represented by -S(R103), and R103 may be selected from the examples of the alkyl group described above.

In this specification, an arylthioxy group may be represented by -S(R104), and R104 may be selected from the examples of the aryl group described above.

In this specification, an alkylsulfoxy group may be represented by -S(=0)2(R105), and R105 may be selected from the examples of the alkyl group described above.

In this specification, an arylsulfoxy group may be represented by -S(=0)2(R106), and R106 may be selected from the examples of the aryl group described above.

In this specification, a heteroaryl group includes at least one heteroatom selected from S, O, Se, N and Si, and may include a monocyclic or polycyclic group having 2 to 60 carbon atoms, and may be further substituted with another substituent. Here, the polycyclic group refers to a group in which a heteroaryl group is directly connected to or fused with another ring group. In this case, the other ring group may be a heteroaryl group, but may also be a different type of ring group, such as a cycloalkyl group, a heterocycloalkyl group, an aryl group, etc. The number of carbon atoms in the heteroaryl group may be 2 to 60, 2 to 40, or 3 to 25. Examples of the heteroaryl group may include a pyridine group, a pyrrole group, a pyrimidine group, a pyridazine group, a furan group, a thiophene group, an imidazole group, a pyrazole group, an oxazole group, an isoxazole group, a thiazole group, an isothiazole group, a triazole group, a furazan group, an oxadiazole group, a thiadiazole group, a dithiazole group, a tetrazolyl group, a pyran group, a thiopyran group, a diazine group, an oxazine group, a thiazine group, a dioxine group, a triazine group, a tetrazine group, a quinoline group, an isoquinoline group, a quinazoline group, an isoquinazoline group, a quinozoline group, a naphthyridine group, an acridine group, a phenanthridine group, an imidazopyridine group, a diazanaphthalene group, a triazaindene group, an indole group, an indolizine group, a benzothiazole group, a benzoxazole group, a benzimidazole group, a benzothiophene group, a benzofuran group, a dibenzothiophene group, a dibenzofuran group, a carbazole group, a benzocarbazole group, a dibenzocarbazole group, a phenazine group, a dibenzosilole group, a spirobi(dibenzosilole), a dihydrophenazine group, a phenoxazine group, phenanthridine group, a thienyl group, an indolo[2,3-a]carbazole group, an indolo[2,3-b]carbazole group, an indoline group, a 10,11-dihydro-dibenzo[b,f]azepine group, a 9,10-dihydroacridine group, a phenanthrazine group, a phenothiathiazine group, a phthalazine group, a phenanthroline group, a naphthobenzofuran group, a naphthobenzothiophene group, a benzo[c][1,2,5]thiadiazole group, a 2,3-dihydrobenzo[b]thiophene group, a 2,3-dihydrobenzofuran group, a 5,10-dihydrodibenzo[b,e][1,4]azacillin group, a pyrazolo[1,5-c]quinazoline group, a pyrido[1,2-b]indazole group, a pyrido[1,2-a]imidazo[1,2-e]indolin group, and a 5,11-dihydroindeno[1,2-b]carbazole group, but are not limited thereto.

In this specification, when the substituent is a carbazole group, it means that the substituent is bonded to either the nitrogen or a carbon atom of the carbazole.

In this specification, when the carbazole group is substituted, an additional substituent may be introduced at either the nitrogen or a carbon atom of the carbazole.

In this specification, examples of the benzocarbazole group may include any of the following structures.

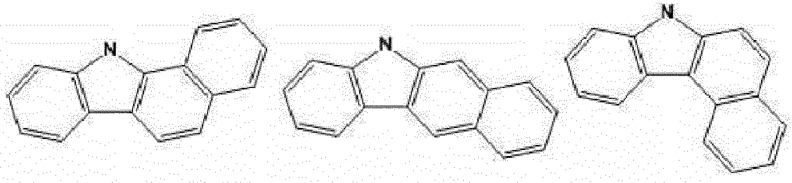

In this specification, examples of the dibenzocarbazole group may be any of the following structures.

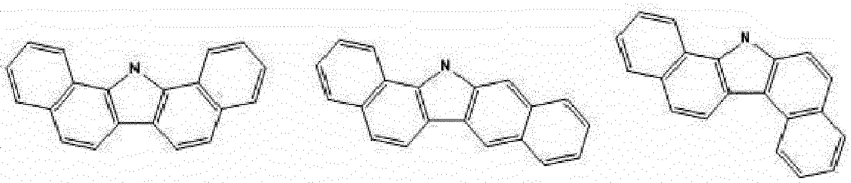

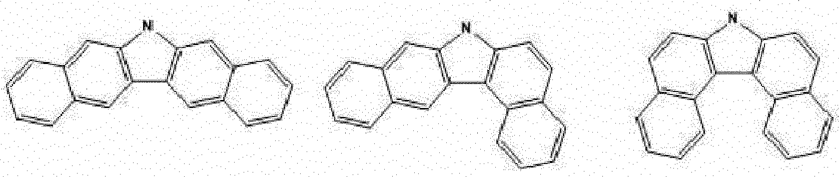

In this specification, examples of the naphthobenzofuran group may be any of the following structures.

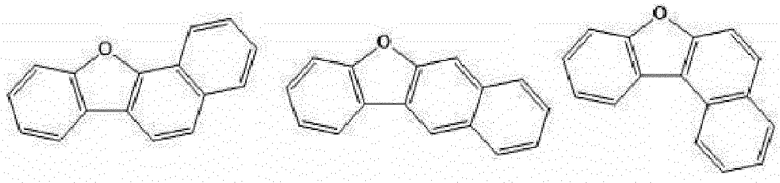

In this specification, examples of the naphthobenzothiophene group may be any of the following structures.

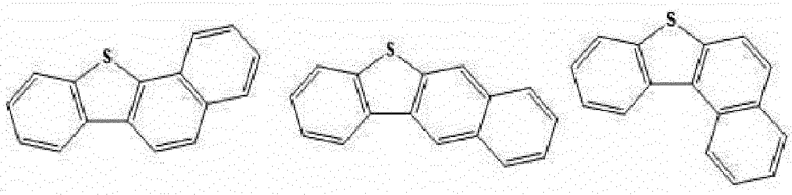

Among the substituents, -SiRR'R" is a silyl group, and is a substituent that includes Si and in which the Si atom is directly connected as a radical. R, R' and R" are the same or different, and may each independently be a substituent consisting of at least one of hydrogen; deuterium; a halogen group; an alkyl group; an alkenyl group; an alkoxy group; a cycloalkyl group; a heterocycloalkyl group; an aryl group; and a heteroaryl group. Examples of the silyl group may include 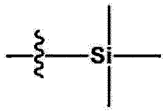 (trimethylsilyl group), 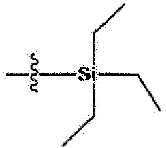 (triethylsilyl group), 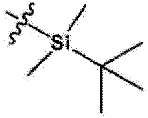 (t-butyldimethylsilyl group), 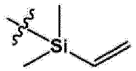 (vinyldimethylsilyl group), 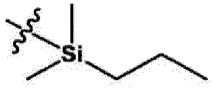 (propyldimethylsilyl group), 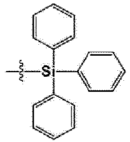 (triphenylsilyl group), 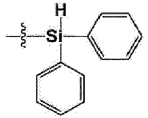 (diphenylsilyl group), 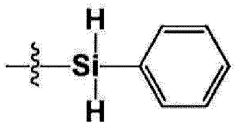 (phenylsilyl group), etc., but are not limited thereto.

Among the substituents, -P(=O)RR' is a phosphine oxide group, wherein R and R' are the same or different, and may each independently be a substituent consisting of at least one of hydrogen; deuterium; a halogen group; an alkyl group; an alkenyl group; an alkoxy group; a cycloalkyl group; a heterocycloalkyl group; an aryl group; and a heteroaryl group, and may be an alkyl group or an aryl group. The alkyl group and the aryl group may be selected from the examples described above. For example, the phosphine oxide group may include a dimethylphosphine oxide group, a diphenylphosphine oxide group, a dinaphthylphosphine oxide group, etc., but is not limited thereto.

Among the substituents, -NRR' is an amine group, and R and R' are the same or different, and may each independently be a substituent consisting of at least one of hydrogen; deuterium; a halogen group; an alkyl group; an alkenyl group; an alkoxy group; a cycloalkyl group; a heterocycloalkyl group; an aryl group; and a heteroaryl group. The amine group may be selected from the group consisting of -NH2; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and the number of carbon atoms is not particularly limited, but is preferably 1 to 30. Examples of the amine group may include a methylamine group, a dimethylamine group, an ethylamine group, a diethylamine group, a phenylamine group, a naphthylamine group, a biphenylamine group, a dibiphenylamine group, anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, and a biphenyltriphenylenylamine group, etc., but are not limited thereto.

In this specification, the arylene group may be selected from the examples of the aryl group described above, except that it is divalent.

In this specification, the heteroarylene group may be selected from the examples of the aryl group described above, except that it is divalent.

In this specification, the "adjacent" group may refer to a substituent substituted on an atom directly bonded to the atom substituted by the corresponding substituent, a substituent stereostructurally positioned closest to the corresponding substituent, or another substituent substituted on the atom bearing the corresponding substituent. For example, two substituents substituted at the ortho position in a benzene ring and two substituents substituted on the same carbon in an aliphatic ring may be interpreted as "adjacent" groups.

Hydrocarbon rings and heterocycles that may be formed by adjacent groups include aliphatic hydrocarbon rings, aromatic hydrocarbon rings, aliphatic heterocycles, and aromatic heterocycles, and the rings may be selected from the structures exemplified as cycloalkyl groups, aryl groups, heterocycloalkyl groups, and heteroaryl groups, except that they are not monovalent.

Generally, compounds bonded with hydrogen and compounds in which hydrogen is substituted with deuterium show differences in thermodynamic behavior. This is because the mass of a deuterium atom is twice that of hydrogen, and deuterium exhibits the characteristic of having lower vibrational energy due to the difference in atomic mass.

In addition, the single bond dissociation energy (BDE) of a carbon-deuterium bond is higher than that of a carbon-hydrogen bond. Therefore, a structure substituted with deuterium exhibits enhanced thermal stability of the molecule, thereby improving the operational lifetime of a device utilizing it.

When a compound is deposited on a silicon wafer, a deuterium-containing compound tends to pack more densely with narrower intermolecular distances. In addition, observation of the thin film surface using an atomic force microscope (AFM) may confirm that a thin film formed from a deuterium-containing compound exhibits a more uniform surface without any aggregation.

The heterocyclic compound of Formula 1 of the present disclosure has a deuterium substitution rate of greater than 0% and less than or equal to 100%. When deuterium is substituted, the ground-state energy becomes lower compared to that of a hydrogen-substituted compound, and as the bond length between carbon and deuterium becomes shorter, the molecular hard-core volume (MHV) decreases. This may reduce the electrical polarizability (EP) and weaken intermolecular interactions, resulting in a more stable stacking structure during device fabrication.

These properties induce a reduction in crystallinity through the formation of an amorphous state in the thin film. In other words, the heterocyclic compound of Formula 1 may be effective in enhancing the heat resistance of OLED devices, which may lead to improvements in both lifetime and operational characteristics.

The heterocyclic compound according to the present disclosure is represented by Formula 1 below.

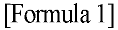

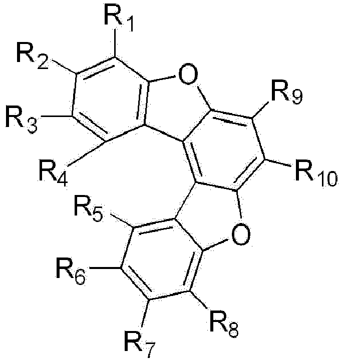

In Formula 1 above, one of R₁ to R₈ is a substituent represented by Formula 2 below, and the others are each independently selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -SiRR'R"; and -P(=O)RR'.

R, R' and R" are each independently hydrogen; deuterium; -CN; a substituted or unsubstituted alkyl group having 1 to 60 carbon atoms; a substituted or unsubstituted cycloalkyl group having 3 to 60 carbon atoms; a substituted or unsubstituted aryl group having 6 to 60 carbon atoms; or a substituted or unsubstituted heteroaryl group having 2 to 60 carbon atoms.

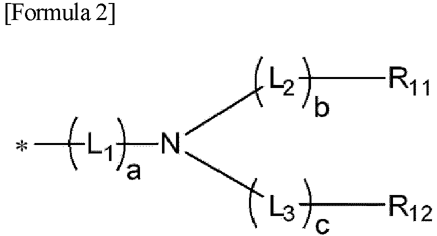

In Formula 2 above, R₁₁ and R₁₂ are each independently a substituted or unsubstituted aryl group having 6 to 60 carbon atoms, or a substituted or unsubstituted heteroaryl group having 2 to 60 carbon atoms.

L₁, L₂ and L₃ are each independently a direct linkage, a substituted or unsubstituted arylene group having 6 to 60 carbon atoms, or a substituted or unsubstituted heteroarylene group having 2 to 60 carbon atoms.

a, b and c are each independently 0 or an integer from 1 to 5.

When a is an integer from 2 to 5, a plurality of L₁ may be the same or different.

When b is an integer from 2 to 5, a plurality of L₂ may be the same or different.

When c is an integer from 2 to 5, a plurality of L₃ may be the same or different.

One of R₉ and R₁₀ may be -L₄-R₁₃, and the other may be hydrogen or deuterium.

L₄ may be a direct linkage, a substituted or unsubstituted arylene group having 6 to 60 carbon atoms, or a substituted or unsubstituted heteroarylene group having 2 to 60 carbon atoms.

R₁₃ may be a substituted or unsubstituted aryl group having 6 to 60 carbon atoms, or a substituted or unsubstituted heteroaryl group having 2 to 60 carbon atoms.

* indicates a bonding site with an adjacent atom.

According to exemplary embodiments, in Formula 1 above, one of R₁ to R₈ may be a substituent represented by Formula 2 above. The substituent represented by Formula 2 above is an arylamine group and may be bonded to the terminal ring of the 5-membered fused ring of Formula 1 above.

According to exemplary embodiments, one of R₁ to R₄ in Formula 1 above may be a substituent represented by Formula 2 above.

According to exemplary embodiments, one of R₅ to R₈ in Formula 1 above may be a substituent represented by Formula 2 above.

According to exemplary embodiments, in Formula 2 above, R₁₁ and R₁₂ may each independently be a substituted or unsubstituted aryl group having 6 to 40 carbon atoms, or a substituted or unsubstituted heteroaryl group having 2 to 40 carbon atoms. According to some embodiments, in Formula 2 above, R₁₁ and R₁₂ may each independently be a substituted or unsubstituted aryl group having 6 to 20 carbon atoms, or a substituted or unsubstituted heteroaryl group having 2 to 15 carbon atoms. R₁₁ and R₁₂ may be the same or different.

For example, in Formula 2 above, R₁₁ and R₁₂ may each independently be a substituted or unsubstituted phenyl group; a substituted or unsubstituted biphenyl group; a substituted or unsubstituted terphenyl group; a substituted or unsubstituted naphthyl group; a substituted or unsubstituted anthryl group; a substituted or unsubstituted dimethyl fluorenyl group; a substituted or unsubstituted diethylfluorenyl group; a substituted or unsubstituted spirobifluorenyl group; a substituted or unsubstituted furan group; a substituted or unsubstituted thiophene; a substituted or unsubstituted benzofuran group; a substituted or unsubstituted benzothiophene group; a substituted or unsubstituted dibenzofuran group; a substituted or unsubstituted dibenzothiophene group; a substituted or unsubstituted carbazole group; or a combination thereof.

For example, in Formula 2 above, R₁₁ and R₁₂ are each independently a phenyl group substituted or unsubstituted with deuterium; a biphenyl group substituted or unsubstituted with deuterium; a terphenyl group substituted or unsubstituted with deuterium; a naphthyl group substituted or unsubstituted with deuterium; a dimethylfluorenyl group substituted or unsubstituted with deuterium; a diphenylfluorenyl group substituted or unsubstituted with deuterium; a spirobifluorenyl group substituted or unsubstituted with deuterium; a furan group substituted or unsubstituted with deuterium; a thiophene group substituted or unsubstituted with deuterium; a benzofuran group substituted or unsubstituted with deuterium; a benzothiophene group substituted or unsubstituted with deuterium; a dibenzofuran group substituted or unsubstituted with deuterium; a dibenzothiophene group substituted or unsubstituted with deuterium; an anthryl group substituted or unsubstituted with deuterium; a carbazole group substituted or unsubstituted with deuterium; or a combination thereof.

According to exemplary embodiments, in Formula 2, L₁, L₂ and L₃ may each independently be a direct linkage, a substituted or unsubstituted arylene group having 6 to 40 carbon atoms, or a substituted or unsubstituted heteroarylene group having 2 to 40 carbon atoms. According to some embodiments, in Formula 2, L₁, L₂ and L₃ may each independently be a direct linkage, a substituted or unsubstituted arylene group having 6 to 20 carbon atoms, or a substituted or unsubstituted heteroarylene group having 2 to 15 carbon atoms. L₁, L₂ and L₃ may be the same or different.

According to exemplary embodiments, in Formula 2 above, L₁, L₂ and L₃ may each independently be a direct linkage, or a substituted or unsubstituted arylene group having 6 to 10 carbon atoms, for example, a substituted or unsubstituted phenylene group, or a substituted or unsubstituted naphthalene group.

According to exemplary embodiments, in Formula 2 above, L₁, L₂ and L₃ may each independently be a direct linkage, an arylene group having 6 to 20 carbon atoms, which may be substituted or unsubstituted with deuterium.

According to exemplary embodiments, L₁, L₂ and L₃ may each independently be a direct linkage, a phenylene group substituted or unsubstituted with deuterium, or a naphthylene group substituted or unsubstituted with deuterium.

According to exemplary embodiments, in Formula 2 above, a, b and c are each independently 0 or an integer from 1 to 5.

In Formula 2 above, when a is an integer from 2 to 5, a plurality of L₁ may be the same or different, when b is an integer from 2 to 5, a plurality of L₂ may be the same or different, and when c is an integer from 2 to 5, a plurality of L₃ may be the same or different.

According to exemplary embodiments, in Formula 2 above, a, b and c may each independently be 0, 1, or 2.

According to exemplary embodiments, Formula 2 above may be represented by any one of Formula 2-1 to Formula 2-8 below.

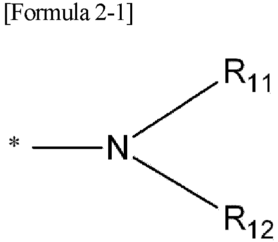

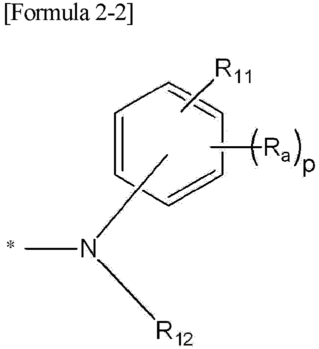

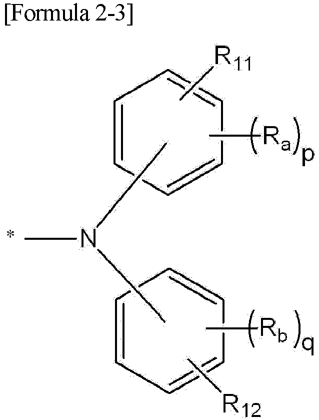

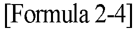

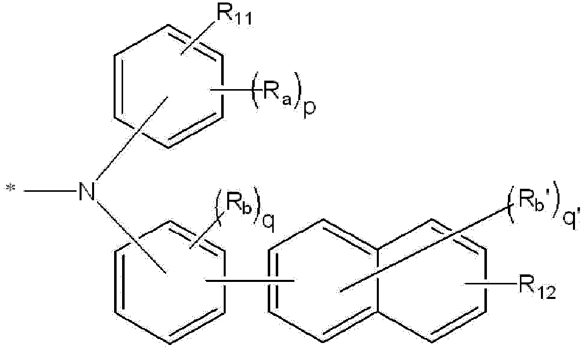

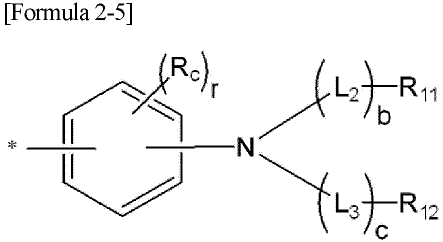

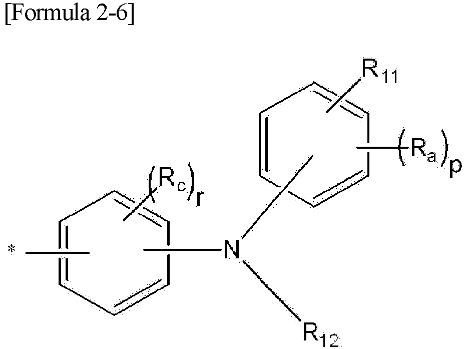

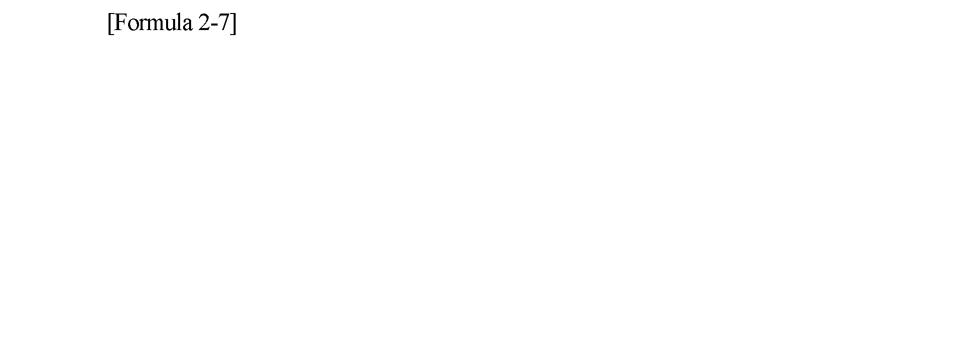

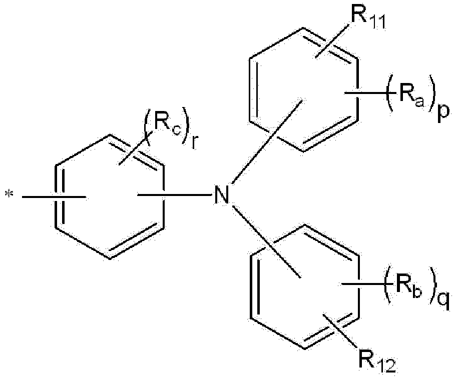

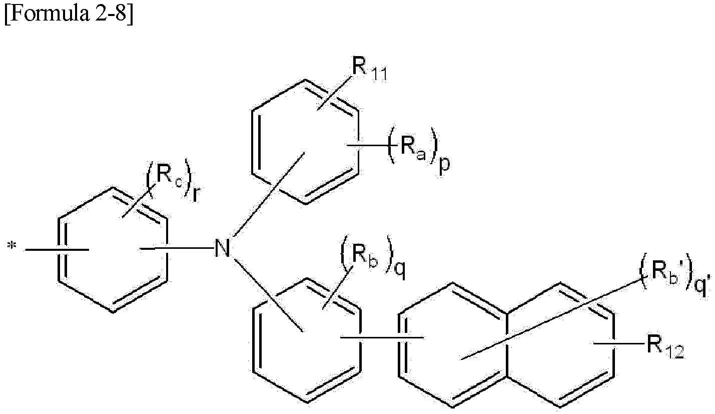

In Formulas 2-1 to 2-8 above, L₂, L₃, b, c, R₁₁ and R₁₂ may be the same as defined in Formula 2 above.

In Formulas 2-1 to 2-8 above, Rₐ, R_{b}, R_{b}' and R_{c} may each be hydrogen or deuterium. p, q and r are each independently 0 or an integer from 1 to 4, and q' may be 0 or an integer from 1 to 8.

According to exemplary embodiments, p may be 0, 3 or 4.

According to exemplary embodiments, q may be 0, 3 or 4.

According to exemplary embodiments, q' may be 0, 1, 2, 3, 7 or 8.

According to exemplary embodiments, r may be 0, 3 or 4.

According to exemplary embodiments, when p, q, q' and r are each independently an integer of 2 or more, a plurality of Rₐ, R_{b}, R_{b}' and R_{c} may each be the same or different.

According to exemplary embodiments, in Formula 1 above, the remaining R₁ to R₈, except the one represented by Formula 2, may each independently be selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; - SiRR'R"; and -P(=O)RR'.

According to exemplary embodiments, in Formula 1 above, one of R₁ to R₄ is a substituent represented by Formula 2 above, and the remaining ones of R₁ to R₄ and R₅ to R₈ may each independently be selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -SiRR'R"; and -P(=O)RR'.

According to exemplary embodiments, in Formula 1 above, one of R₅ to R₈ is a substituent represented by Formula 2 above, and the remaining ones of R₅ to R₈ and R₁ to R₄ may each independently be selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -SiRR'R"; and -P(=O)RR'.

According to exemplary embodiments, in Formula 1 above, the remaining R₁ to R₈, except the one represented by Formula 2, may each independently be hydrogen or deuterium.

According to exemplary embodiments, in Formula 1 above, the remaining R₁ to R₄, except the one represented by Formula 2, and R₅ to R₈ may each independently be hydrogen or deuterium.

According to exemplary embodiments, in Formula 1 above, the remaining R₅ to R₈, except the one represented by Formula 2, and R₁ to R₄ may each independently be hydrogen or deuterium.

According to exemplary embodiments, in Formula 1 above, one of R₉ and R₁₀ is - (L₄)m-R₁₃, and the other is hydrogen or deuterium.

In exemplary embodiments, R₉ may be -(L₄)n-R₁₃, and R₁₀ may be hydrogen or deuterium.

In exemplary embodiments, R₁₀ may be -(L₄)n-R₁₃, and R₉ may be hydrogen or deuterium.

n may be an integer from 1 to 5. In some embodiments, n may be 1 or 2. For example, when n is an integer from 2 to 5, a plurality of L₄ may be the same or different.

L₄ may be a direct linkage, a substituted or unsubstituted arylene group having 6 to 60 carbon atoms, or a substituted or unsubstituted heteroarylene group having 2 to 60 carbon atoms.

In exemplary embodiments, L₄ may be a direct linkage, a substituted or unsubstituted arylene group having 6 to 40 carbon atoms, or a substituted or unsubstituted heteroarylene group having 2 to 40 carbon atoms.

In exemplary embodiments, L₄ may a direct linkage, a substituted or unsubstituted arylene group having 6 to 20 carbon atoms, or a substituted or unsubstituted heteroarylene group having 2 to 15 carbon atoms.

In exemplary embodiments, L₄ may be a direct linkage; a substituted or unsubstituted phenylene group; or a substituted or unsubstituted biphenylene group.

In exemplary embodiments, L₄ may be a direct linkage; a phenylene group substituted or unsubstituted with deuterium; or a biphenylene group substituted or unsubstituted with deuterium.

The R₁₃ is a substituted or unsubstituted aryl group having 6 to 60 carbon atoms, or a substituted or unsubstituted heteroaryl group having 2 to 60 carbon atoms.

In exemplary embodiments, R₁₃ may be a substituted or unsubstituted aryl group having 6 to 40 carbon atoms, or a substituted or unsubstituted heteroaryl group having 2 to 40 carbon atoms.

In exemplary embodiments, R₁₃ may be a substituted or unsubstituted aryl group having 6 to 20 carbon atoms, or a substituted or unsubstituted heteroaryl group having 2 to 15 carbon atoms.

In exemplary embodiments, R₁₃ may be a substituted or unsubstituted phenyl group; a substituted or unsubstituted naphthyl group; a substituted or unsubstituted biphenyl group; a substituted or unsubstituted anthracene group; a substituted or unsubstituted triphenylene group; or a combination thereof.

In exemplary embodiments, R₁₃ may be a phenyl group substituted or unsubstituted with deuterium; a naphthyl group substituted or unsubstituted with deuterium; a biphenyl group substituted or unsubstituted with deuterium; an anthracene group substituted or unsubstituted with deuterium; a triphenylene group substituted or unsubstituted with deuterium; or a combination thereof.

In exemplary embodiments, one of R₉ and R₁₀ may be represented by any one of Formulas 3-1 to 3-6 below, and the other may be hydrogen or deuterium.

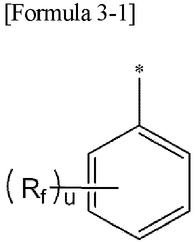

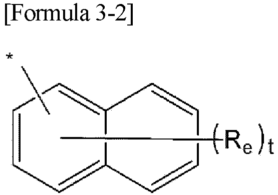

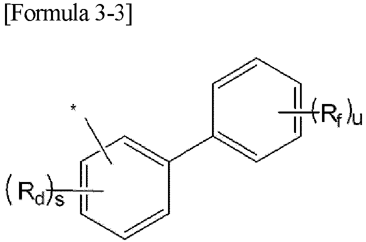

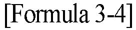

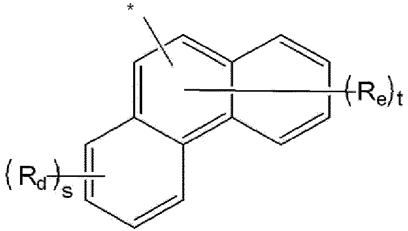

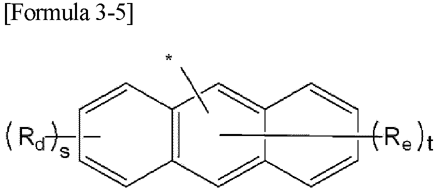

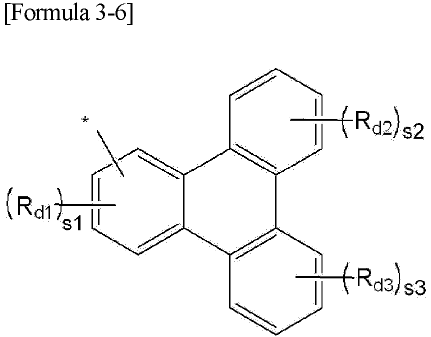

In Formulas 3-1 to 3-6 above, R_{d}, R_{d1}, R_{d2}, R_{d3}, Rₑ and R_{f} are each hydrogen or deuterium.

In Formulas 3-1 to 3-6, s may be 0 or an integer from 1 to 4. s1 may be 0 or an integer from 1 to 3, and s2 and s3 may each independently be 0 or an integer from 1 to 4. t may be 0 or an integer from 1 to 5. u may be 0 or an integer from 1 to 5.

According to exemplary embodiments, s may be 0, 1, 2, 3 or 4.

According to exemplary embodiments, s1 may be 0, 1, 2 or 3.

According to exemplary embodiments, s2 may be 0, 1, 2, 3 or 4.

According to exemplary embodiments, s3 may be 0, 1, 2, 3 or 4.

According to exemplary embodiments, t may be 0, 1, 2, 3, 4 or 5.

According to exemplary embodiments, u may be 0, 1, 2, 3, 4 or 5.

According to exemplary embodiments, when s, s1, s2, s3, t and u are each independently an integer of 2 or more, a plurality of R_{d}, R_{d1}, R_{d2}, R_{d3}, Rₑ and R_{f} may be the same or different.

According to exemplary embodiments, one of R₁ to R₄ is a substituent represented by Formula 2 above, and the remaining ones of R₁ to R₄ and R₅ to R₈ may each independently be hydrogen or deuterium. R₉ may be -L₄-R₁₃, and R₁₀ may be hydrogen or deuterium. The heterocyclic compound may include an arylamine group bonded to a terminal ring located closer to the carbon atom bonded to the substituent on the third ring of the 5-membered fused structure is bonded. Accordingly, the intermolecular interaction may be smoother, and the band gap may be controlled to achieve a desired value.

In exemplary embodiments, the heterocyclic compound may be represented by Formula 1-1 below.

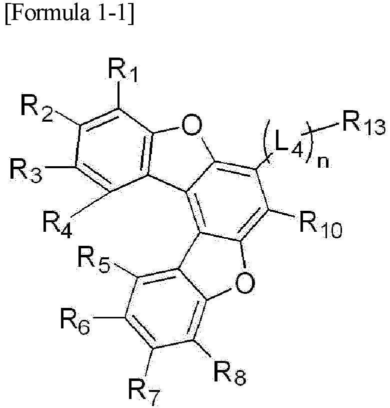

In Formula 1-1 above, one of R₁ to R₄ is a substituent represented by Formula 2, and the others may each independently be hydrogen or deuterium.

R₅ to R₈ and R₁₀ may each independently be hydrogen or deuterium.

L₄ may be a direct linkage, a substituted or unsubstituted arylene group having 6 to 60 carbon atoms, or a substituted or unsubstituted heteroarylene group having 2 to 60 carbon atoms.

n is an integer from 1 to 5, and when n is an integer from 2 to 5, a plurality of L₄ may be the same or different.

R₁₃ may be a substituted or unsubstituted aryl group having 6 to 60 carbon atoms, or a substituted or unsubstituted heteroaryl group having 2 to 60 carbon atoms.

In exemplary embodiments, the heterocyclic compound may include one nitrogen atom in the molecule. In some embodiments, the heterocyclic compound may not include a nitrogen atom other than the nitrogen atom included in Formula 2.

For example, one of R₁ to R₈ of Formula 1 above may be a substituent represented by Formula 2 above, and the remaining ones of R₁ to R₈, as well as R₉ and R₁₀ may not include a nitrogen atom.

For example, the heterocyclic compound may not include a nitrogen-containing heteroaryl group.

Accordingly, the carrier balance of the heterocyclic compound may be improved, and the capacitance characteristics due to the excess electrons may be effectively realized.

According to exemplary embodiments, the deuterium content of the compound of Formula 1 may be 0%, or greater than 0 and less than or equal to 100%.

According to exemplary embodiments, the deuterium content of the compound of Formula 1 may be 0%, or 5% to 100%.

According to exemplary embodiments, the deuterium content of the compound of Formula 1 may be 0%, or 10% to 100%.

According to exemplary embodiments, the deuterium content of the compound of Formula 1 may be 0%, or 15% to 100%.

According to exemplary embodiments, the deuterium content of the compound of Formula 1 may be 0%, or 20% to 100%.

According to exemplary embodiments, the deuterium content of the compound of Formula 1 may be 0%, or 25% to 100%.

According to exemplary embodiments, the deuterium content of the compound of Formula 1 may be 0%, or 30% to 100%.

According to exemplary embodiments, the deuterium content of the compound of Formula 1 may be 0%, or 50% to 100%.

According to exemplary embodiments, the deuterium content of the compound of Formula 1 may be 0%, or 70% to 100%.

According to exemplary embodiments, the deuterium content of the compound of Formula 1 may be 0%, or 90% to 100%.

According to exemplary embodiments, the deuterium content of the compound of Formula 1 may be 0%.

According to exemplary embodiments, the deuterium content of the compound of Formula 1 may be 30% to 100%.

According to exemplary embodiments, the deuterium content of the compound of Formula 1 may be 50% to 100%.

According to exemplary embodiments, the deuterium content of the compound of Formula 1 may be 70% to 100%.

According to exemplary embodiments, the deuterium content of the compound of Formula 1 may be 90% to 100%.

The heterocyclic compound may be represented by any one of the chemical formulas selected from the following.

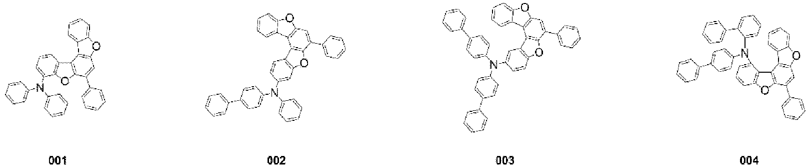

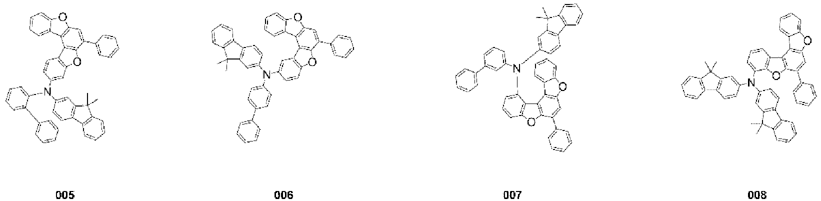

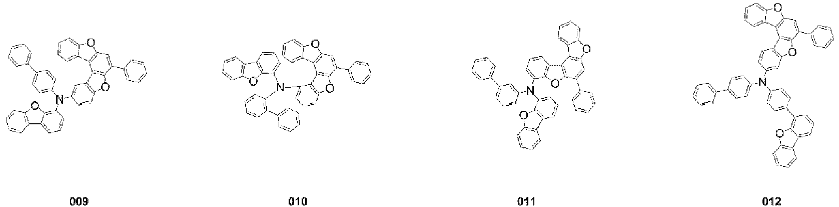

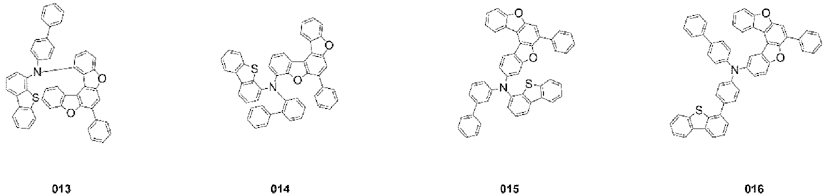

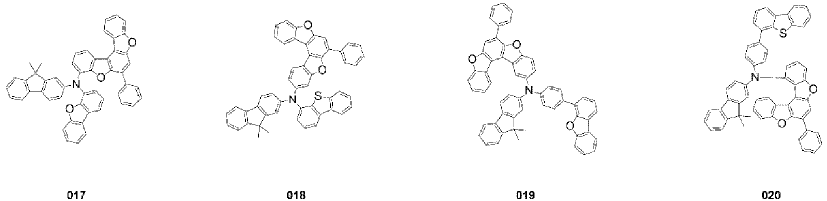

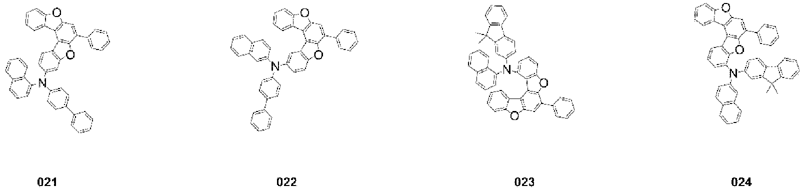

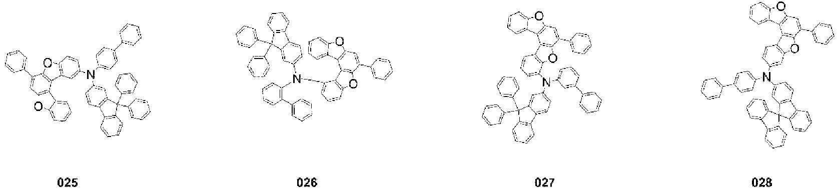

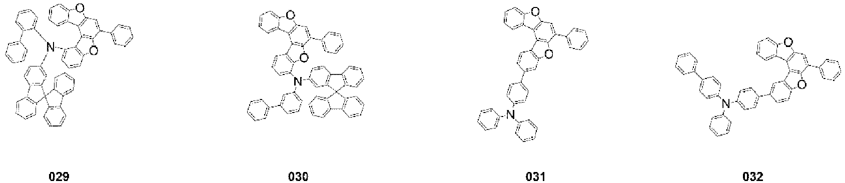

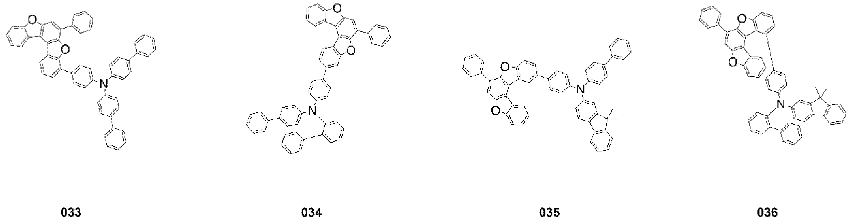

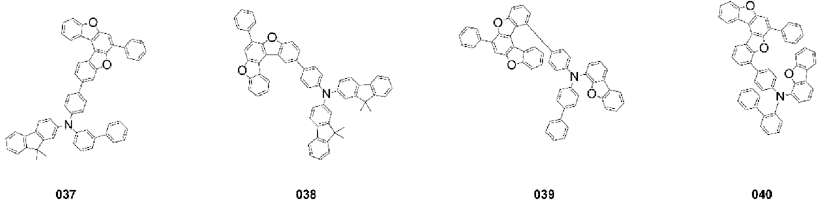

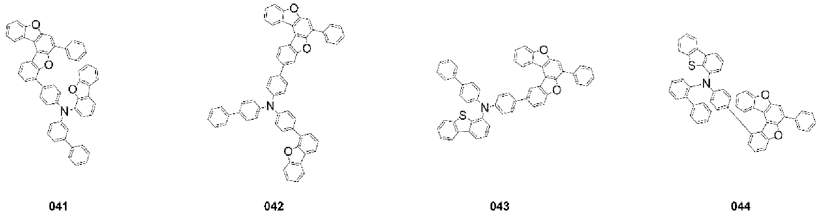

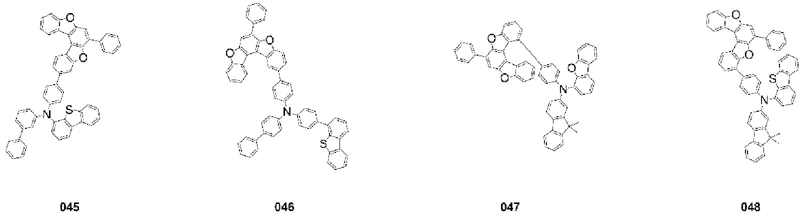

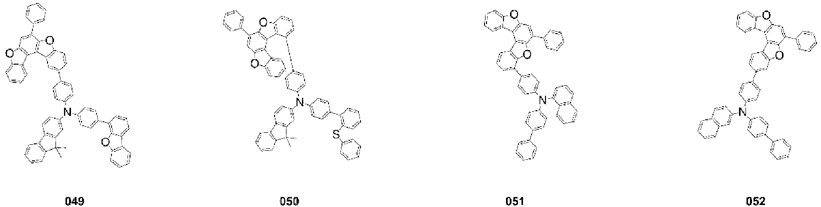

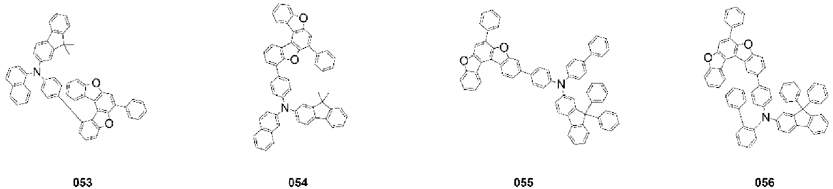

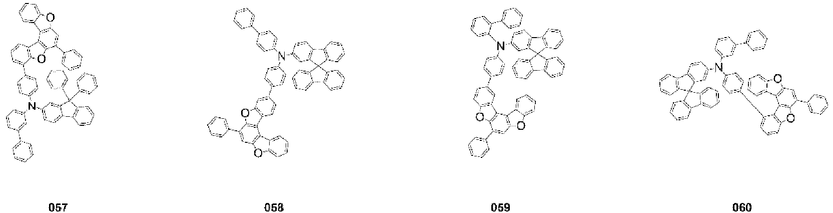

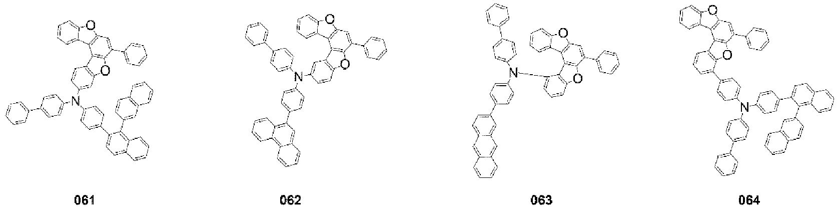

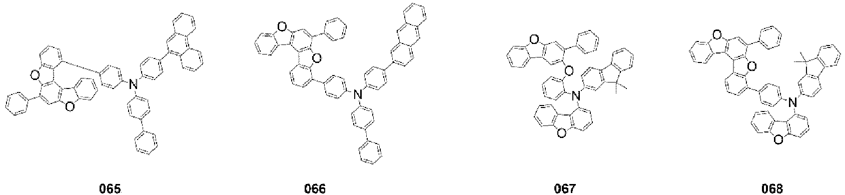

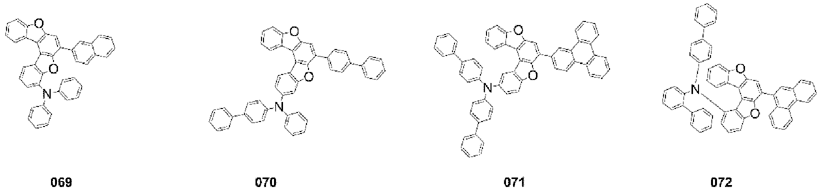

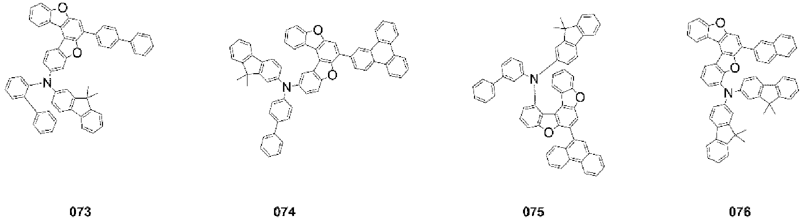

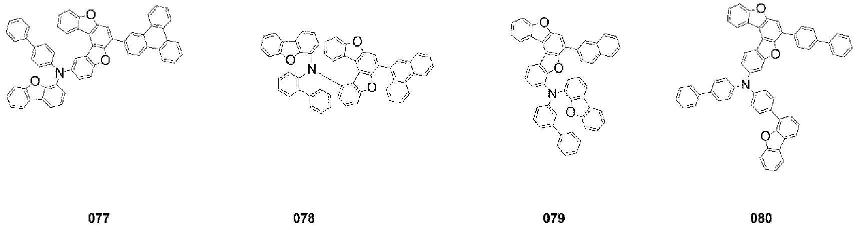

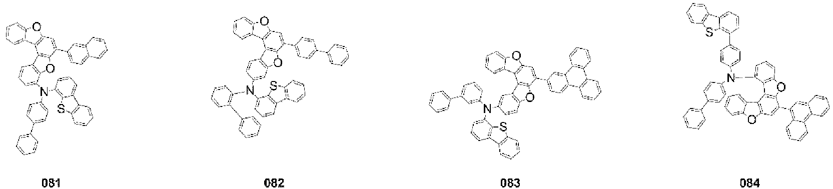

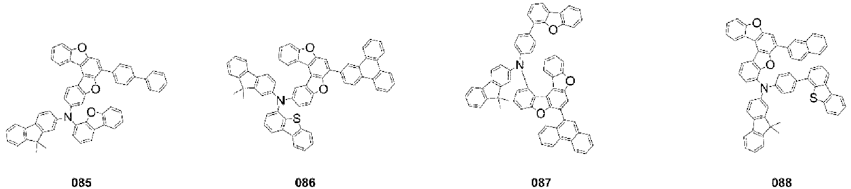

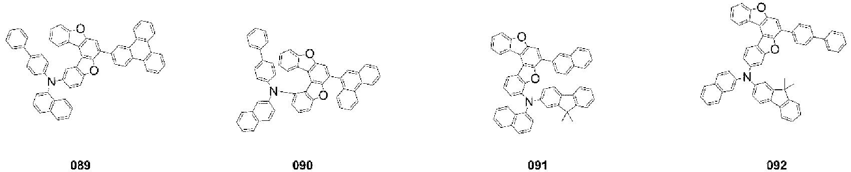

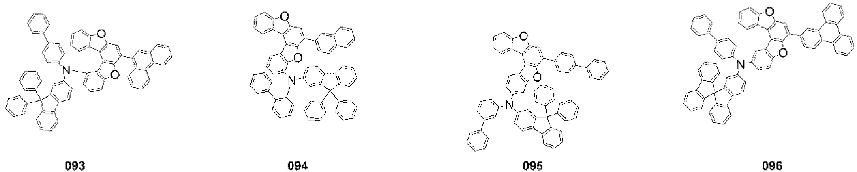

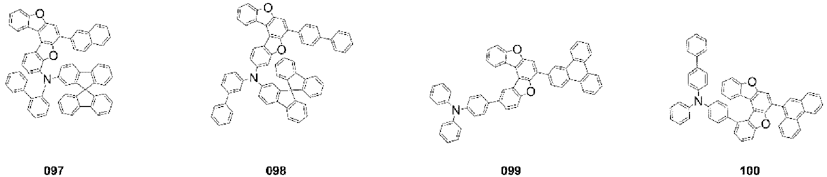

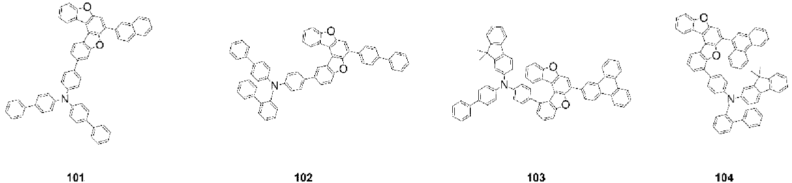

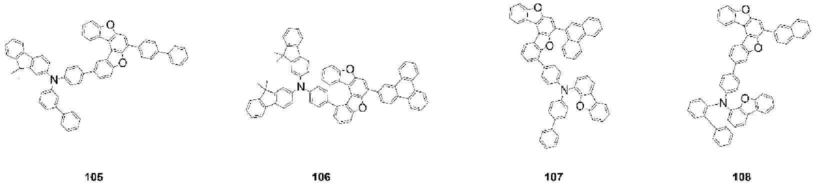

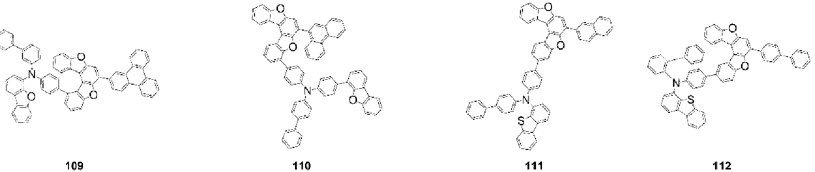

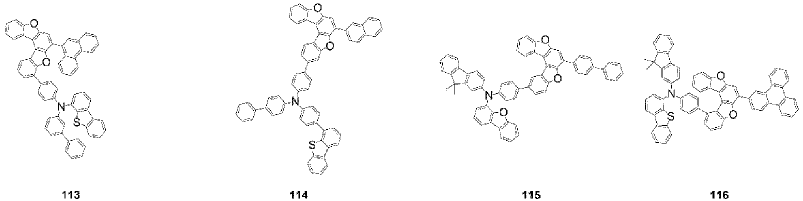

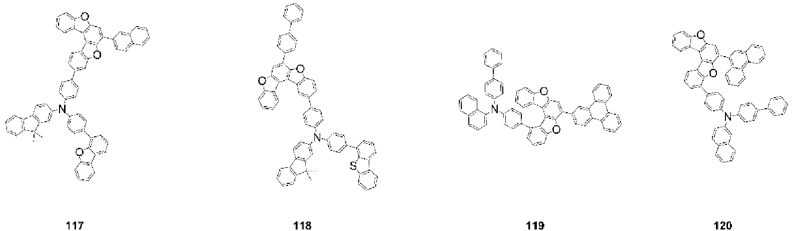

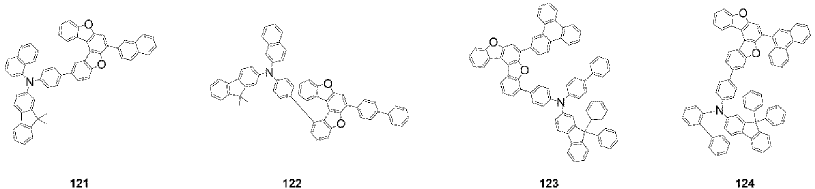

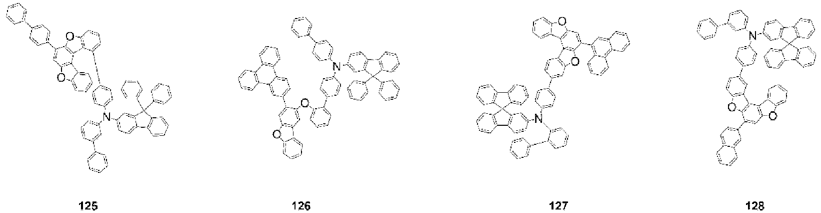

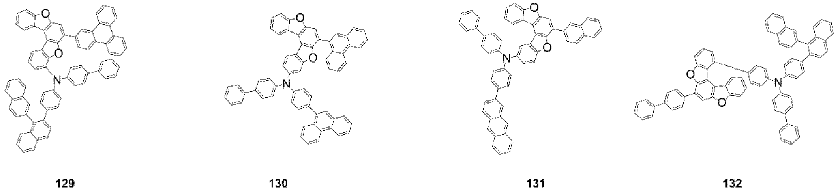

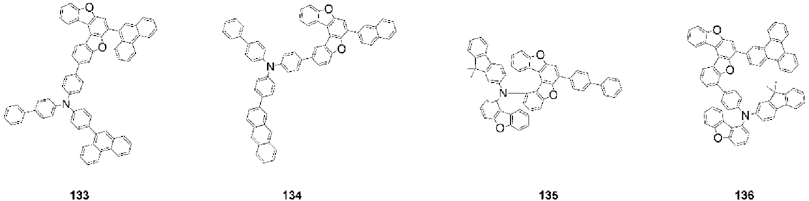

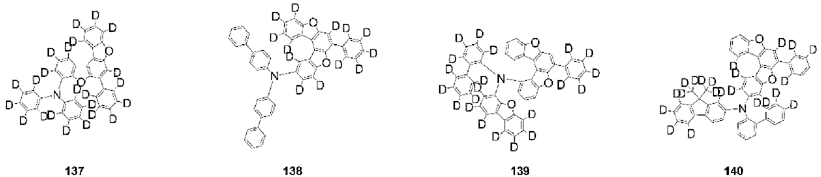

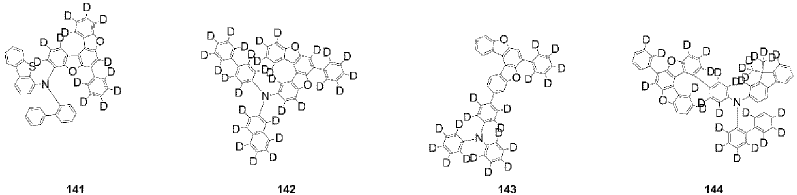

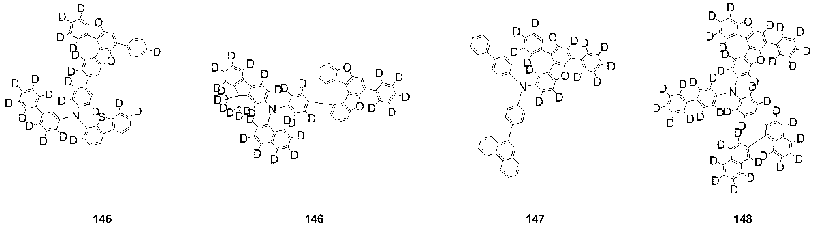

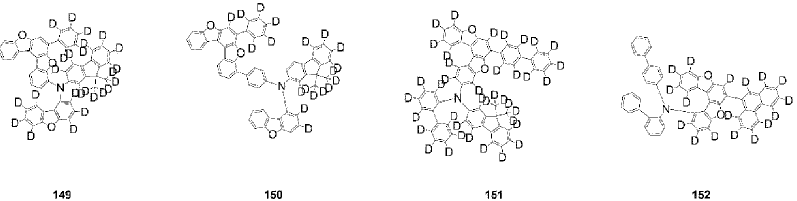

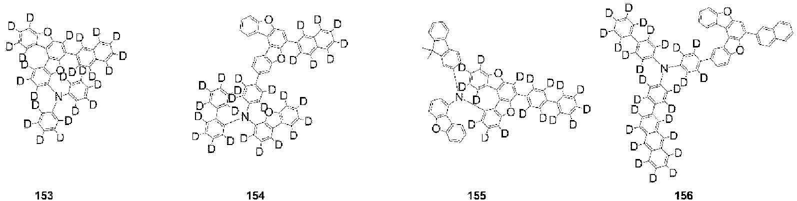

In one embodiment of the present disclosure, there is provided an organic light-emitting device including: a first electrode; a second electrode provided opposite to the first electrode; and one or more organic layers provided between the first electrode and the second electrode. One or more of the organic layers include a heterocyclic compound represented by Formula 1 above.

In an exemplary embodiment, one or more of the organic layers may include one type of the heterocyclic compound represented by Formula 1 above.

In an exemplary embodiment, one or more of the organic layers may include two or more types of the heterocyclic compound represented by Formula 1 above.

In an exemplary embodiment, the first electrode may be a cathode, and the second electrode may be an anode. Alternatively, the first electrode may be an anode, and the second electrode may be a cathode.

In an exemplary embodiment, the organic light-emitting device may be a blue organic light-emitting device, and the heterocyclic compound according to Formula 1 above may be used as a material for the blue organic light-emitting device.

In an exemplary embodiment, the organic light-emitting device may be a green organic light-emitting device, and the heterocyclic compound represented by Formula 1 may be used as a material for the green organic light-emitting device.

In an exemplary embodiment, the organic light-emitting device may be a red organic light-emitting device, and the heterocyclic compound represented by Formula 1 may be used as a material for the red organic light-emitting device.

In this specification, the specific details of the heterocyclic compound represented by Formula 1 are the same as those described above.

The organic light-emitting device may be fabricated by a conventional fabrication methods and materials for an organic light-emitting device, except that one or more organic layers are formed using the above-described heterocyclic compound.

The heterocyclic compound may be formed into an organic layer not only by a vacuum deposition method but also by a solution application method when fabricating the organic light-emitting device. Here, the solution application method refers to spin coating, dip coating, inkjet printing, screen printing, spray coating, roll coating, etc., but is not limited thereto.

The organic layer of the organic light-emitting device may be formed as a single-layer structure, but may also be formed as a multilayer structure in which two or more organic layers are stacked. For example, the organic light-emitting device may have a structure including a hole injection layer, a hole transport layer, a light-emitting layer, an electron transport layer, an electron injection layer, etc. as the organic layers. However, the structure of the organic light-emitting device is not limited thereto and may include fewer or more organic layers.

The organic layer of the organic light-emitting device may include a light-emitting layer. The light-emitting layer may include the heterocyclic compound.

The light-emitting layer may include a host material, and the host material may include the heterocyclic compound.

According to exemplary embodiments, the organic layer of the organic light-emitting device may include a hole transport layer (HTL), an auxiliary hole transport layer (prime HTL), and an electron blocking layer (EBL), and at least one of the hole transport layer, the auxiliary hole transport layer, and the electron blocking layer may include a heterocyclic compound represented by Formula 1 above.

According to exemplary embodiments, the organic layer may include a hole transport layer (HTL) and an auxiliary hole transport layer, and at least one of the hole transport layer and the auxiliary hole transport layer may include the heterocyclic compound.

According to exemplary embodiments, the organic layer may include an electron blocking layer (EBL), and the electron blocking layer may include the heterocyclic compound.

According to exemplary embodiments, the organic layer may include a hole transport layer, a light-emitting layer, or an electron blocking layer, and the hole transport layer, the light-emitting layer, or the electron blocking layer may include the heterocyclic compound.

According to exemplary embodiments, the organic layer may include a hole transport layer or an auxiliary hole transport layer, and the hole transport layer or the auxiliary hole transport layer may include the compound.

According to exemplary embodiments, the organic layer may further include a light-emitting auxiliary layer or an N-type charge generation layer (N-CGL).

According to exemplary embodiments, materials having a relatively large work function, such as transparent conductive oxides, metals, or conductive polymers, may be used as the cathode material. Examples of the cathode materials include metals such as vanadium, chromium, copper, zinc, and gold, or alloys thereof; metal oxides such as zinc oxide, indium oxide, indium tin oxide (ITO), and indium zinc oxide (IZO); combinations of metals and oxides such as ZnO:Al or SnO2:Sb; and conductive polymers such as poly(3-methylthiophene), poly[3,4-(ethylene-1,2-dioxy)thiophene] (PEDOT), polypyrrole and polyaniline, but are not limited thereto.

Materials having relatively low work functions may be used as anode materials, including metals, metal oxides, or conductive polymers. Examples of the anode materials may include metals such as magnesium, calcium, sodium, potassium, titanium, indium, yttrium, lithium, gadolinium, aluminum, silver, tin, and lead, or alloys thereof, as well as multilayer structures such as LiF/Al or LiO₂/Al, but are not limited thereto.

As the hole injection layer material, known materials for preparing a hole injection layer may be used. For example, phthalocyanine compounds such as copper phthalocyanine disclosed in U.S. Patent No. 4,356,429, or starburst-type amine derivatives described in the literature [Advanced Material, 6, p. 677 (1994)] may be used. Examples thereof may include tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), and 4,4',4"-tris(N,N-(2-naphthyl)-phenylamino)triphenylamine (2-TNATA). Soluble conductive polymers may also be used, such as polyaniline/dodecylbenzenesulfonic acid, poly(3,4-ethylenedioxythiophene)/poly(4-styrenesulfonate), polyaniline/camphor sulfonic acid, or polyaniline/poly(4-styrene-sulfonate).

As the hole transport layer material, pyrazoline derivatives, arylamine derivatives, stilbene derivatives, triphenyldiamine derivatives, etc. may be used. Both low-molecular-weight and high-molecular-weight materials may also be used. As the electron transport layer material, oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, etc. may be used. Both low-molecular-weight and high-molecular-weight materials may also be used.

As the electron injection layer material, for example, LiF is commonly used in the art, but the present application is not limited thereto.

As the light-emitting material, red, green, or blue light-emitting materials may be used, and if necessary, two or more types of light-emitting materials may be mixed and used. In this case, the two or more types of light-emitting materials may be deposited using separate sources, or may be premixed and deposited using a single source. In addition, a fluorescent material may be used as the light-emitting material, or a phosphorescent material may also be used. As the light-emitting material, a material that emits light by combining holes and electrons injected from the anode and cathode, respectively, may be used individually, but materials in which both a host material and a dopant material participate in light emission may also be used.

When using a mixture of hosts for the light-emitting material, hosts of the same series may be mixed and used, or hosts of different series may also be mixed and used. For example, two or more materials selected from n-type host materials or p-type host materials may be used as host materials for the light-emitting layer.

According to exemplary embodiments, the organic light-emitting device may be a front-emitting, back-emitting, or dual-sided light-emitting device depending on the material used.

According to exemplary embodiments, the heterocyclic compound may function in organic electronic devices, including organic solar cells, organic photoconductors, and organic transistors, based on principles similar to those applied to the organic light-emitting devices.

The organic light-emitting device may further include one or more layers selected from the group consisting of a light-emitting layer, a hole injection layer, a hole transport layer, an electron injection layer, an electron transport layer, an electron blocking layer and a hole blocking layer.

The organic light-emitting device may further include one or more layers selected from the group consisting of a light-emitting layer, a hole injection layer, a hole transport layer, an electron blocking layer, a hole blocking layer, a charge generation layer, an electron transport layer and an electron injection layer.

FIGS. 1 to 3 illustrate the stacking order of the electrodes and organic layers of the organic light-emitting device according to an embodiment of the present application. However, it is not intended that the scope of the present application be limited to these drawings, and structures of organic light-emitting devices known in the art may also be applicable to the present application.

Referring to FIG. 1, an organic light-emitting device in which a cathode 200, an organic layer 300, and an anode 400 are sequentially stacked on a substrate 100 is illustrated. However, the structure is not limited thereto, and an organic light-emitting device in which an anode, an organic layer, and a cathode are sequentially stacked on a substrate, as shown in FIG. 2, may also be employed.

FIG. 3 illustrates an example in which the organic layer has a multilayer structure.

The organic light-emitting device according to FIG. 3 includes a hole injection layer 301, a hole transport layer 302, a light-emitting layer 303, an electron transport layer 304 and an electron injection layer 305. However, the scope of the present application is not limited to such a stacked structure, and one or more of the layers other than the light-emitting layer may be omitted, or additional functional layers may be further included.

The organic layer including the heterocyclic compound of Formula 1 may further include other materials as needed.

According to an exemplary embodiment, a method for fabricating the organic light-emitting device may be provided.

According to exemplary embodiments, a substrate may be prepared. A first electrode may be formed on the substrate. One or more organic layers may be formed on the first electrode. A second electrode may be formed on the organic layer to fabricate the organic light-emitting device.

The organic layer may be formed using an organic layer composition including a heterocyclic compound represented by Formula 1 above.

Hereinafter, embodiments of the present invention will be further described with reference to specific experimental examples. However, the following examples and comparative examples included in the experimental examples are only given for illustrating the present invention and those skilled in the art will obviously understand that various alterations and modifications are possible within the scope and spirit of the present invention. Such alterations and modifications are duly included in the appended claims.

### Preparative Example 1

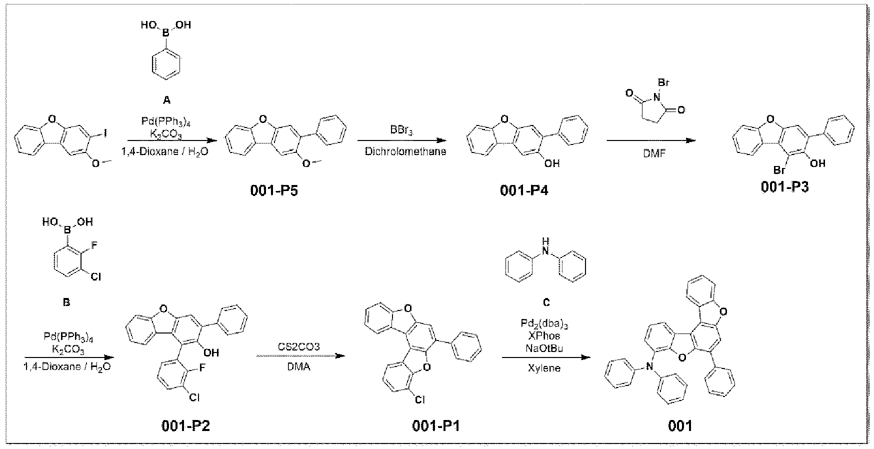

### 1) Preparation of Compound 001-P5

3-iodo-2-methoxydibenzofuran (20 g, 60.47 mmol, 1 equivalent "eq"), phenylboronic acid (A) (9.03 g, 72.57 mmol, 1.2 eq), potassium carbonate (K₂CO₃) (20.89 g, 151.18 mmol, 2.5 eq), and tetrakis(triphenylphosphine)palladium (Pd(PPh₃)₄) (3.49 g, 3.02 mmol, 0.05 eq) were added to a mixed solvent of 1,4-dioxane (200 mL) and deionized water (70 mL), and the mixture was stirred at 100 °C for 6 h. Then, moisture was removed, and the resulting product was purified by silica gel column chromatography to obtain 10 g of Compound 001-P5 with a yield of 60.28%.

### 2) Preparation of Compound 001-P4

Compound 001-P5 (10 g, 36.46 mmol, 1 eq) and boron tribromide (BBr₃) (5.19 ml, 54.68 mmol, 1.5 eq) were added to dichloromethane (100 mL), and the mixture was stirred at room temperature for 1 h. The organic layer was extracted using dichloromethane and water, and moisture was then removed with magnesium sulfate (MgSO₄). After purification by silica gel column chromatography, 7.4 g of Compound 001-P4 was obtained with a yield of 77.9%.

### 3) Preparation of Compound 001-P3

Compound 001-P4 (7.4 g, 36.46 mmol, 1 eq) and N-bromosuccinimide (NBS) (5.57 g, 31.27 mmol, 1.1 eq) were added to dimethylformamide (DMF) (74 ml), and the mixture was stirred at 80°C for 4 h. After completion of the reaction, deionized water (Di water) (74 ml) was added. The precipitated solid was filtered to remove the solvent and residual moisture, and 7.2 g of Compound 001-P3 was obtained with a yield of 74.67%.

### 4) Preparation of Compound 001-P2

Compound 001-P3 (7.2 g, 21.23 mmol, 1 eq), 3-chloro-2-fluorophenylboronic acid (B) (4.07 g, 23.35 mmol, 1.1 eq), potassium carbonate (K₂CO₃) (20.89 g, 151.18 mmol, 2.5 eq), and tetrakis(triphenylphosphine)palladium (Pd(PPh₃)₄) (3.49 g, 3.02 mmol, 0.05 eq) were added to a mixed solvent of 1,4-dioxane (200 mL) and deionized water (70 mL), and the mixture was stirred at 100°C for 6 h. After removal of moisture, the resulting product was purified by silica gel column chromatography to obtain 7 g of Compound 001-P2 with a yield of 84.81%.

### 5) Preparation of Compound 001-P1

Compound 001-P2 (7 g, 18 mmol, 1 eq) and cesium carbonate (Cs₂CO₃) (11.73 g, 36.01 mmol, 2 eq) were added to N,N-dimethylacetamide (DMA) (110 mL), and the mixture was stirred at 150°C for 3 h. The organic layer was extracted using methylene chloride (MC) and water, and moisture was then removed with magnesium sulfate (MgSO₄). After purification by silica gel column chromatography, 6.1 g of Compound 001-P1 was obtained with a yield of 91.8%.

### 6) Preparation of Compound 001

Compound 001-P1 (6.1 g, 16.54 mmol, 1 eq), N-phenylaniline (C) (2.8 g, 16.54 mmol, 1 eq), tris(dibenzylideneacetone)dipalladium (Pd₂(dba)₃) (0.76 g, 0.83 mmol, 0.05 eq), Xphos (0.79 g, 1.65 mmol, 0.1 eq), sodium tert-butoxide (NaOtBu) (3.97 g, 41.35 mmol, 2.5 eq), and toluene (6.1 ml) were mixed and stirred at 100°C for 1 h. After completion of the reaction, the mixture was concentrated and purified by silica gel column chromatography to obtain 7 g of Compound 001 with a yield of 84.3 8%.

A compound was synthesized in the same manner as in the above preparative example, by using intermediate A listed in Table 1 below instead of phenylboronic acid (A) in step 1), intermediate B listed in Table 1 below instead of 3-chloro-2-fluorophenylboronic acid (B) in step 3), and intermediate C listed in Table 1 below instead of N-phenylalanine (C) in step 6).

**[TABLE 1]**

| Compound No. | Intermediate A | Intermediate B | Intermediate C | Yield |
|---|---|---|---|---|
| 003 | 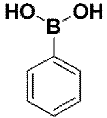 | 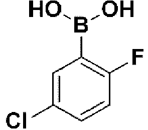 | 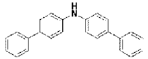 | 67% |
| 005 | 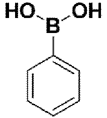 | 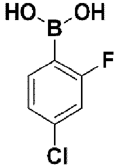 | 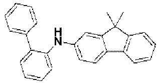 | 71% |
| 008 | 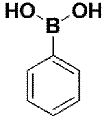 | 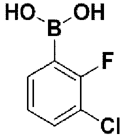 | 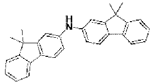 | 56% |
| 014 | 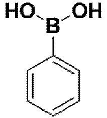 | 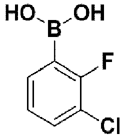 | 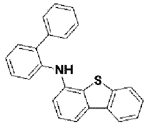 | 66% |
| 017 | 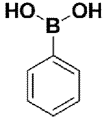 | 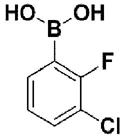 | 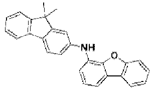 | 60% |
| 023 | 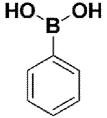 | 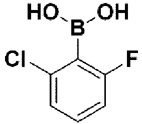 | 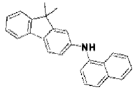 | 51% |
| 026 | 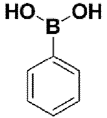 | 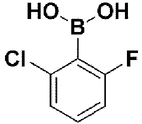 | 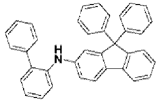 | 74% |
| 061 | 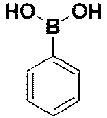 | 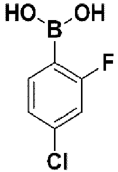 | 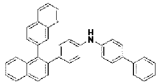 | 51% |
| 067 | 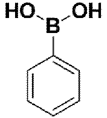 | 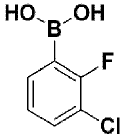 | 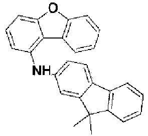 | 42% |
| 073 | 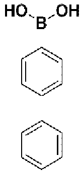 | 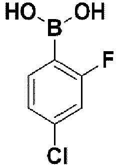 | 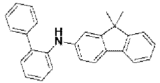 | 51% |
| 078 | 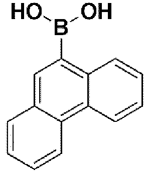 | 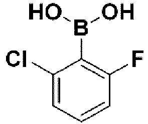 | 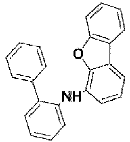 | 66% |
| 082 | 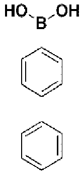 | 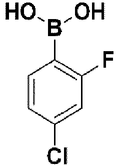 | 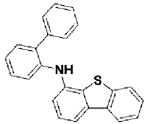 | 51% |
| 086 | 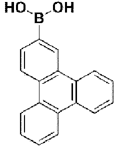 | 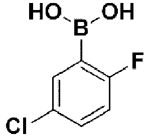 | 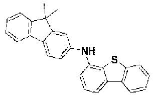 | 62% |
| 093 | 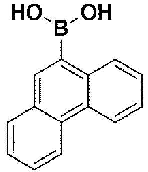 | 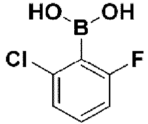 | 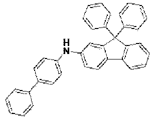 | 71% |
| 129 | 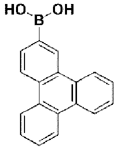 | 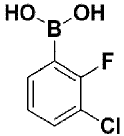 | 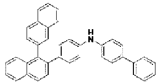 | 51% |
| 135 | 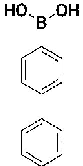 | 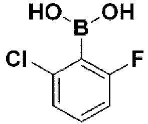 | 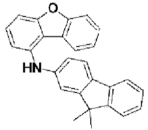 | 55% |

### Preparative Example 2

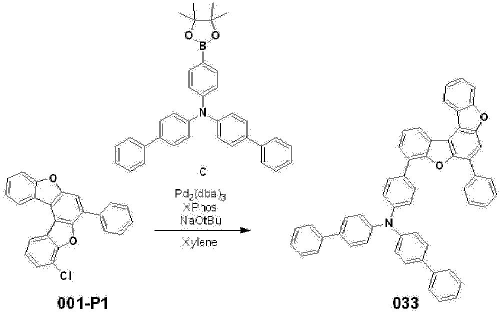

Compound 001-P1 (10 g, 27.11 mmol, 1 eq) from step 5) of Preparative Example1, 4,4,5,5-tetramethyl-N,N-bis(4-phenylphenyl)-1,3,2-dioxaborolan-2-amine (C) (12.13 g, 27.11 mmol, 1 eq), tetrakis(triphenylphosphine)palladium (Pd(PPh₃)₄) (1.57 g, 1.36 mmol, 0.05 eq), and potassium carbonate (K₂CO₃) (9.37 g, 67.79 mmol, 2.5 eq) were added to a mixed solvent of 1,4-dioxane (100 mL) and water (30 mL), and the mixture was stirred at 100°C for 6 h. The organic layer was extracted using methylene chloride (MC) and water, and moisture was then removed with magnesium sulfate (MgSO₄). The resulting product was purified by silica gel column chromatography to obtain 13.5 g of Compound 033 with a yield of 76.16%.

A compound was synthesized in the same manner as in Preparative Example 1, by using intermediate A listed in Table 2 below instead of phenylboronic acid (A) in step 1), intermediate B listed in Table 2 below instead of 3-chloro-2-fluorophenylboronic acid (B) in step 3), and intermediate C listed in Table 2 below instead of 4,4,5,5-tetramethyl-N,N-bis(4-phenylphenyl)-1,3,2-dioxaborolan-2-amine (C) in step 6).

**[TABLE 2]**

| Compound No. | Intermediate A | Intermediate B | Intermediate C | Yield |
|---|---|---|---|---|
| 034 | 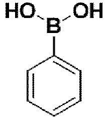 | 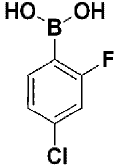 | 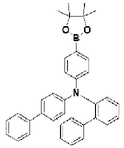 | 68% |
| 036 | 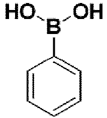 | 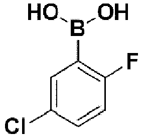 | 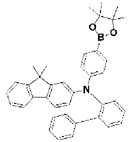 | 72% |
| 038 | 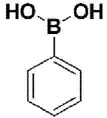 | 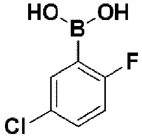 | 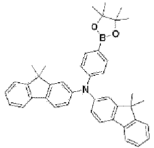 | 55% |
| 044 | 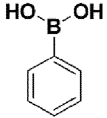 | 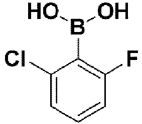 | 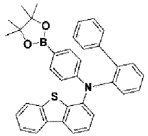 | 61% |
| 048 | 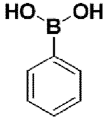 | 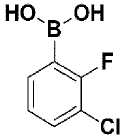 | 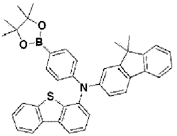 | 71% |
| 059 | 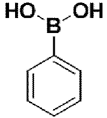 | 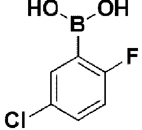 | 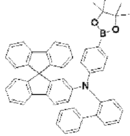 | 66% |
| 064 | 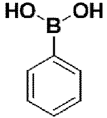 | 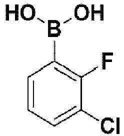 | 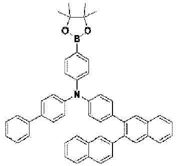 | 76% |
| 068 | 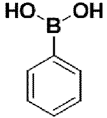 | 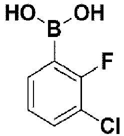 | 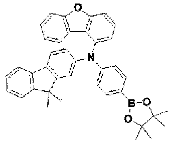 | 74% |
| 099 | 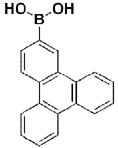 | 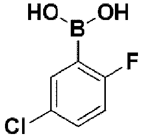 | 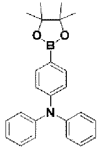 | 67% |
| 104 | 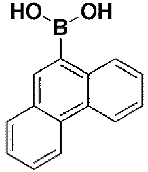 | 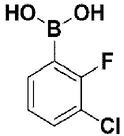 | 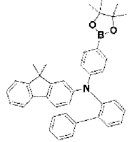 | 46% |
| 112 | 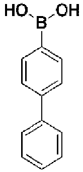 | 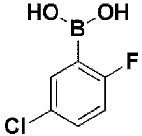 | 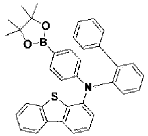 | 51% |
| 113 | 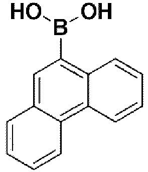 | 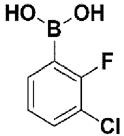 | 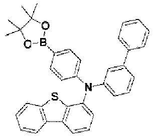 | 71% |
| 120 | 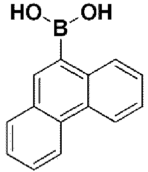 | 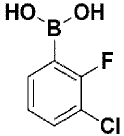 | 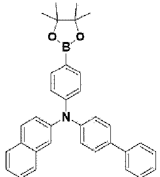 | 75% |
| 127 | 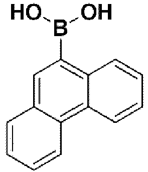 | 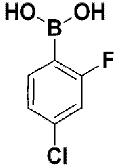 | 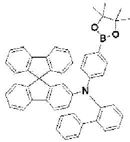 | 81% |
| 133 | 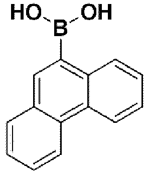 | 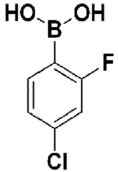 | 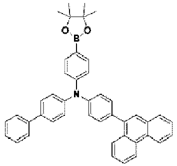 | 77% |
| 136 | 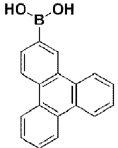 | 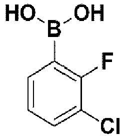 | 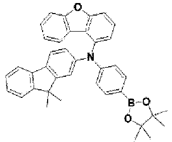 | 66% |

### Preparative Example 3

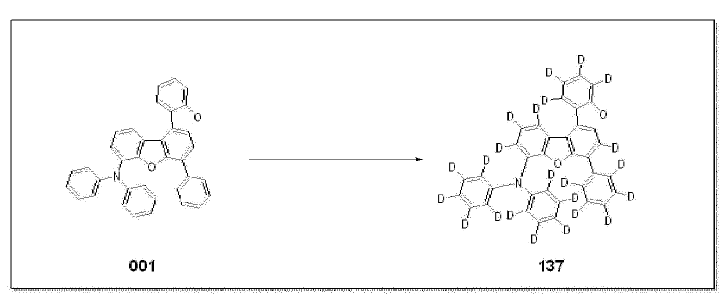

Compound 001 (5 g, 9.97 mmol) was added to 50 mL of C₆D₆ and purged with nitrogen for 2 hours to prepare a reaction mixture. Trifluoromethanesulfonic acid (69.78 mmol, 7 eq) (6.27 mL) was added dropwise to the reaction mixture using a syringe, and the mixture was refluxed for 2 hours.

After cooling to room temperature, 50 mL of deuterated water was added for extraction. The organic layer was dried over anhydrous magnesium sulfate (MgSO₄) and concentrated using a rotary evaporator. Then, Compound 137 (3.7 g, 70.7%) was obtained from a slurry in ethylene acetate (EA).

A compound was synthesized in the same manner as in Preparative Example 3, except that the reaction compound, reaction temperature and time, and the equivalent of trifluoromethanesulfonic acid were changed as shown in Table 3 below.

**[TABLE 3]**

| Prepared compound No. | Reaction Compound No. | Reaction temperature | Reaction time | Trifluoromethane sulfonic acid equivalent | Yield |
|---|---|---|---|---|---|
| 138 | 003 | Room temperature (No reflux) | 2 HR | 5 eq | 66 % |
| 139 | 010 | Room temperature (No reflux) | 5 HR | 5 eq | 59 % |
| 140 | 005 | Reflux | 2 HR | 5 eq | 61 % |
| 144 | 036 | Reflux | 2 HR | 5 eq | 64 % |
| 149 | 067 | Reflux | 2 HR | 5 eq | 64 % |
| 150 | 068 | Room temperature (No reflux) | 5 HR | 5 eq | 70 % |
| 154 | 108 | Room temperature (No reflux) | 5 HR | 5 eq | 70 % |
| 155 | 135 | Reflux | 2 HR | 5 eq | 53 % |

The compounds in Tables 1 to 3 were analyzed by ¹H NMR (DMSO, 300 MHz) and field desorption mass spectrometry (FD-MS) to confirm their synthesis, and the results are presented in Tables 4 and 5, respectively.

**[TABLE 4]**

| Compoun d No. | 1H NMR(DMSO, 300MHz) |
|---|---|
| 1 | 7.98(1H, d), 7.64(1H, d), 7.54~7.24(14H, m), 7.08~6.97(7H, m) |
| 3 | 822(1H, s), 7.98(1H, d), 7.75(4H, d), 7.56~7.31(24H, m), 6.97(1H, d) |
| 5 | 8.10(1H, d), 8.03~7.98(2H, m), 7.90~7.80(3H, d), 7.55~7.28(18H, m), 7.16~7.08(4H, m), 6.91(1H, d), 1.69(6H, s) |
| 8 | 7.98(1H, d), 7.90~7.86(4H, d), 7.64(1H, d), 7.55~7.28(18H, ,m), 7.16(2H, d), 6.97(1H, d) 1.69(12H, s) |
| 14 | 8.45(1H, d), 8.11~8.10(2H, d), 7.98~7.93(2H, d), 7.56~7.28(20H, m), 7.14~7.08(3H, m), 6.97(1H, d) |
| 17 | 7.98(2H, d), 7.90~7.86(2H, d), 7.64(2H, d), 7.55~7.28(18H, m), 7.16(1H, d), 6.97(2H, d), 1.69(6H, s) |
| 23 | 8.22~8.15(2H, d), 7.98(1H, d), 7.90~7.81(3H, d), 7.63~7.28(19H, m), 7.16(1H, d), 6.91(1H, d), 1.69(6H, s) |
| 26 | 8.10(1H, d), 7.98(1H, d), 7.90~7.86(2H, d), 7.54~7.10(34H, m), 6.91(1H, d) |
| 33 | 8.08~7.98(3H, d), 7.75(4H, d), 7.55~7.31(28H, m) |
| 34 | 8.10(1H, d), 8.03~7.98(2H, d), 7.82~7.75(4H, m), 7.55~7.31(25H, m), 7.14~7.08(3H, m) |
| 36 | 8.10(1H, d), 7.98(1H, d), 7.90~7.82(3H, d), 7.69(1H, d), 7.57~7.28(23H, m), 7.16~7.08(4H, m) 1.69(6H, s) |
| 38 | 7.98(1H, d), 7.90~7.79(7H, m), 7.55~7.28(21H. m), 7.16(2H, d), 1.69(12H, s) |
| 44 | 8.45(1H, d), 8.11~8.10(2H, d), 7.98~7.93(2H, d), 7.82(1H, d), 7.69(1H, d), 7.57~7.31(23H, m), 7.14~7.08(3H, m) |
| 48 | 8.45(1H, d), 8.11~7.86(7H, d), 7.56~7.28(19H, m), 7.16(1H, d), 1.69(6H, s) |
| 59 | 8.10(1H, d), 7.98(1H, d), 7.90~7.79(7H, m), 7.55~7.23(28H, m), 7.14~7.06(4H, m) |
| 61 | 8.93(1H, d), 8.31~8.26(2H, d), 8.09~7.98(5H, m), 7.80~7.72(4H, d), 7.63~7.31(26H, m), 6.91(1H, d) |
| 64 | 8.93(1H, d), 8.31~8.26(2H, d), 8.09~7.98(6H, d), 7.75~7.72(3H, d), 7.63~7.31(31H, m) |
| 67 | 7.98(2H, d), 7.90~7.86(2H, d), 7.64(1H, d), 7.55~7.25(19H, m), 7.16(1H, d), 6.97~6.91(2H, d), 1.69(6H, s) |
| 68 | 8.08~7.98(4H, d), 7.90~7.86(2H, d), 7.55~7.25(23H, m), 7.16(1H, d), 6.91(1H, d), 1.69(6H, s) |
| 73 | 8.10(1H, d), 8.03~7.98(2H, m), 7.86~7.75(4H, d), 7.55~7.25(20H, m), 7.16~7.08(4H, m), 6.91(1H, d), 1.69(6H, s) |
| 78 | 9.08(1H, d), 8.84(1H, d), 8.27(1H, d), 8.10~7.90(5H, m), 7.70~7.53(8H, m), 7.43~7.25(12H, m), 7.14~7.08(3H, m), 6.97~6.91(2H, d) |
| 82 | 8.45(1H, d), 8.11~8.10(2H, d), 8.03~7.93(3H, m), 7.80~7.75(3H, d), 7.56~7.25(20H, m), 7.14~7.08(3H, m), 6.91(1H, d) |
| 86 | 9.27(1H, s), 8.79(1H, d), 8.45(1H, d), 8.37~8.30(4H, d), 8.22(1H, s), 8.11(1H, d), 7.98~7.86(4H, d), 7.70~7.31(18H, m), 7.16(1H, d), 6.97(1H, d), 1.69(6H, s) |
| 93 | 9.08(1H, d), 8.84(1H, d), 8.27(1H, d), 8.05(1H, s), 7.98~7.86(4H, d), 7.75~7.10(34H, m), 6.91(1H, d) |
| 99 | 9.27(1H, s), 8.79(1H, d), 8.37~8.30(4H, d), 7.98(1H, d), 7.88~7.52(12H, m), 7.39~7.24(8H, m), 7.08~7.00(6H, m) |
| 104 | 9.08(1H, d), 8.84(1H, d), 8.27(1H, d), 8.10~7.98(5H, m), 7.90~7.86(2H, d), 7.70~7.28(22H, m), 7.16~7.08(4H, m), 1.69(6H, s) |
| 112 | 8.45(1H, d), 8.11~8.10(2H, d), 7.98~7.75(7H, d), 7.56~7.25(24H, m), 7.14~7.08(3H, m) |
| 113 | 9.08(1H, d), 8.84(1H, d), 8.45(1H, d), 8.27(1H, d), 8.11~7.90(7H, m), 7.75~7.27(24H, m), 7.18~7.17(2H, d) |
| 120 | 9.08(1H, d), 8.84(1H, d), 8.27(1H, d), 8.08~7.98(4H, m), 7.90(1H, d), 7.78~7.31(28H, m), 7.11(1H, s) |
| 127 | 9.08(1H, d), 8.84(1H, d), 8.27(1H, d), 8.10~7.23(38H, m), 7.14~7.06(4H, m) |
| 129 | 9.27(1H, s), 8.93(1H, d), 8.79(1H, d), 8.37~8.26(6H, d), 8.09~7.98(4H, d), 7.75~7.28(31H, m), 6.97(1H, d) |
| 133 | 9.08(1H, d), 8.84(1H, d), 8.27(2H, d), 8.05~7.98(4H, m), 7.90(2H, d), 7.82~7.31(31H, m) |
| 135 | 7.98(2H, d), 7.90~7.86(2H, d), 7.75(2H, d), 7.55~7.25(22H, m), 7.16(1H, d), 6.91(2H, d), 1.69(6H, s) |
| 136 | 9.27(1H, s), 8.79(1H, d), 8.37~8.30(4H, d), 8.08~7.98(4H, d), 7.90~7.86(2H, d), 7.70~7.51(12H, m), 7.39~7.25(11H, m), 7.16(1H, d), 6.91(1H, d), 1.69(6H, s) |
| 137 | Fully D-substituted compound |
| 138 | 7.75(4H, d), 7.55~7.37(14H, m) |
| 139 | 7.98(1H, d), 7.54~7.53(2H, m), 7.39~7.25(4H, m), 6.97(1H, d) |
| 140 | 8.10(1H, d), 7.86(1H, d), 7.77(1H, d), 7.59~7.52(3H, m), 7.43~7.36(5H, m), 7.14(1H, t), 6.94(1H, d) |
| 144 | 7.90~7.86(2H, d), 7.77(1H, d), 7.60~7.52(3H, m), 7.43~7.42(2H, t), 7.16(1H, t), 7.06(1H, t), 6.94(1H, d) |
| 149 | 7.98(1H, d), 7.64(1H, d), 7.54~7.53(2H, m), 7.39~7.28(3H, t), 6.97(1H, d) |
| 150 | 8.08~7.98(4H, d), 7.86(1H, d), 7.55~7.51(5H, m), 7.39~7.31(7H, m), 6.94(1H, d) |
| 155 | 7.98(1H, d), 7.90~7.86(2H, d), 7.55~7.54(2H, d), 7.39~7.25(7H, m), 7.16(1H, d), 6.91(1H, d), 1.69(6H, s) |

**[TABLE 5]**

| Compoun d No. | FD-MS | Compound No. | FD-MS |
|---|---|---|---|
| 001 | m/z= 501.17 (C36H23NO2 =501.58) | 003 | m/z= 653.24 (C48H31NO2 =653.78) |
| 005 | m/z= 693.27 (C51H35NO2 =693.85) | 008 | m/z= 733.30 (C54H39NO2 =733.91) |
| 014 | m/z= 683.19(C48H29NO2S =683.83) | 017 | m/z= 707.25 (C51H33NO3 = 707.83) |
| 023 | m/z= 667.25 (C49H33NO2 =667.81) | 026 | m/z= 817.30 (C61H39NO2 =817.99) |
| 033 | m/z= 729.27 (C54H35NO2 =729.88) | 034 | m/z= 729.27 (C54H35NO2 =729.88) |
| 036 | m/z= 769.30(C57H39NO2 =769.94) | 038 | m/z= 809.33 (C60H43NO2 = 810.01) |
| 044 | m/z= 759.22(C54H33NO2S = 759.92) | 048 | m/z= 799.25 (C57H37NO2S =799.99) |
| 059 | m/z= 891.31 (C67H41NO2 = 892.07) | 061 | m/z= 829.30 (C62H39NO2 =830.00) |
| 064 | m/z= 905.33 (C68H43NO2 = 906.10) | 067 | m/z= 707.25 (C51H33NO3 =707.83) |
| 068 | m/z= 783.28(C57H37NO3= 783.93) | 073 | m/z= 769.30 (C57H39NO2= 769.94) |
| 078 | m/z= 767.25 (C56H33NO3= 767.88) | 082 | m/z= 759.22 (C54H33NO2S= 759.92) |
| 086 | m/z= 873.27(C63H39NO2S= 874.07) | 093 | m/z= 917.33 (C69H43NO2= 918.11) |
| 099 | m/z= 727.25(C54H33NO2= 727.86) | 104 | m/z= 869.33 (C65H43NO2= 870.06) |
| 112 | m/z= 835.25 (C60H37NO2S= 836.02) | 113 | m/z= 859.25 (C62H37NO2S= 860.04) |
| 120 | m/z= 803.28(C60H37NO2 = 803.96) | 127 | m/z= 991.35 (C75H45NO2= 992.19) |
| 129 | m/z= 979.35(C74H45NO2= 980.18) | 133 | m/z= 929.33 (C70H43NO2 = 930.12) |
| 135 | m/z= 783.28(C57H37NO3 = 783.93) | 136 | m/z= 933.32 (C69H43NO3 = 934.11) |
| 137 | m/z= 524.32(C36D23NO2 = 524.73) | 138 | m/z= 666.32 (C48H18D13NO2 = 666.86) |
| 139 | m/z= 688.35(C48H8D21NO3 = 688.89) | 140 | m/z= 715.40 (C51H13D22NO2 = 715.98) |
| 144 | m/z= 797.47 (C57H11D28NO2 = 798.11) | 149 | m/z= 732.40 (C51H8D25NO3 = 732.98) |
| 150 | m/z= 802.40(C57H18D19NO3 = 803.04) | 154 | m/z= 820.43 (C58H8D27NO3 = 821.09) |
| 155 | m/z= 800.38 (C57H20D17NO3 = 801.03) | | |

### Fabrication of Organic light-emitting device 1

A transparent ITO thin-film electrode on an OLED glass substrate (fabricated by Samsung-Coming) was ultrasonically cleaned for 5 minutes each using trichloroethylene, acetone, ethanol, and distilled water in sequence, and then stored in isopropanol until use. Next, the substrate was installed in the substrate holder of the vacuum deposition equipment, and 4,4',4"-tris(N,N-(2-naphthyl)-phenylamino)triphenyl amine (2-TNATA) was loaded into the crucible of the vacuum deposition equipment.

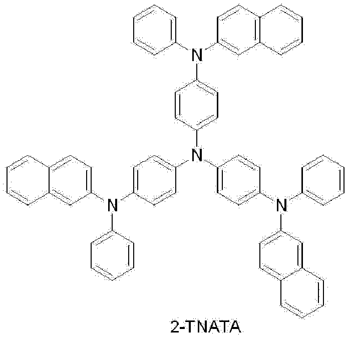

Then, after the vacuum inside the chamber was evacuated until it reached 10⁻⁶ torr, a current was applied to the crucible to evaporate 2-TNATA and deposit a hole injection layer having a thickness of 600 Å on the ITO thin-film electrode (cathode).

A compound of Formula 001 was placed in another crucible in the vacuum deposition equipment, and a current was applied to the crucible to evaporate it and deposit a hole transport layer having a thickness of 600 Å on the hole injection layer.

A blue light-emitting material was deposited on the hole transport layer by the following method to form a light-emitting layer. Specifically, a blue light-emitting host material, a compound of Structural Formula H1 below, was vacuum-deposited to a thickness of 200 Å in one crucible in the vacuum deposition equipment, and a blue light-emitting dopant material, a compound of Structural Formula D1 below, was vacuum-deposited thereon in an amount of 5 parts by weight based on 100 parts by weight of the blue light-emitting host material.

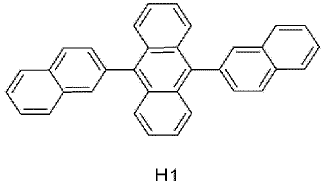

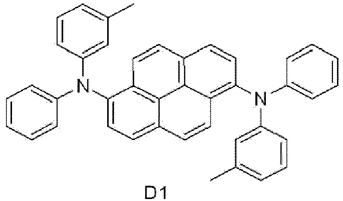

A compound of Structural Formula E1 below was vacuum-deposited to a thickness of 300 Å on the light-emitting layer to form an electron transport layer.

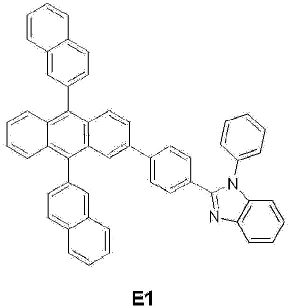

Lithium fluoride (LiF) was deposited to a thickness of 10 Å on the electron transport layer as an electron injection layer, and an OLED device was fabricated with an Al anode having a thickness of 1,000 Å.

All organic compounds required for the fabrication of the OLED device were purified by vacuum sublimation at 10⁻⁶ to 10⁻⁸ torr for each material and used in the device fabrication.

An organic light-emitting device was fabricated in the same manner as described above, except that the compound of Formula 001 used in the formation of the hole transport layer was replaced with the compounds listed in Table 6 below.

### Experimental Example 1

The electroluminescence (EL) characteristics of the organic light-emitting device were measured using the M7000 system from MacScience. Based on the measurement results, the T₉₅ value was determined using the M6000 lifetime measurement system from MacScience at a reference luminance of 700 cd/m².

The measured results for the operating voltage, luminous efficiency, color coordinates (CIE), and lifetime of the organic light-emitting device are shown in Table 6 below.

**[TABLE 6]**

| | Compound No. | Operating voltage (V) | Luminous efficiency (cd/A) | CIE (x, y) | Lifetime (T₉₅) |
|---|---|---|---|---|---|
| Example 1 | 001 | 4.84 | 6.79 | (0.133, 0.100) | 86 |
| Example 2 | 003 | 4.87 | 6.83 | (0.134, 0.102) | 91 |
| Example 3 | 005 | 4.82 | 6.88 | (0.133, 0.100) | 88 |
| Example 4 | 014 | 4.83 | 6.86 | (0.133, 0.101) | 80 |
| Example 5 | 017 | 4.86 | 6.05 | (0.133, 0.100) | 86 |
| Example 6 | 033 | 4.84 | 6.21 | (0.134, 0.101) | 88 |
| Example 7 | 034 | 4.81 | 6.29 | (0.133, 0.100) | 88 |
| Example 8 | 036 | 4.85 | 6.82 | (0.133, 0.100) | 89 |
| Example 9 | 044 | 4.87 | 6.81 | (0.133, 0.100) | 86 |
| Example 10 | 048 | 4.86 | 6.79 | (0.133, 0.101) | 83 |
| Example 11 | 061 | 4.86 | 6.82 | (0.133, 0.100) | 81 |
| Example 12 | 067 | 4.81 | 6.90 | (0.134, 0.101) | 89 |
| Example 13 | 068 | 4.82 | 6.86 | (0.133, 0.100) | 81 |
| Example 14 | 073 | 4.85 | 6.79 | (0.133, 0.100) | 81 |
| Example 15 | 082 | 4.72 | 6.82 | (0.133, 0.100) | 83 |
| Example 16 | 093 | 4.81 | 6.90 | (0.133, 0.100) | 89 |
| Example 17 | 104 | 4.86 | 6.86 | (0.133, 0.100) | 81 |
| Example 18 | 112 | 4.88 | 6.83 | (0.133, 0.101) | 81 |
| Example 19 | 129 | 4.79 | 6.82 | (0.134, 0.100) | 83 |
| Example 20 | 135 | 4.84 | 6.81 | (0.133, 0.100) | 83 |
| Example 21 | 136 | 4.86 | 6.79 | (0.133, 0.100) | 89 |
| Example 22 | 137 | 4.90 | 6.82 | (0.133, 0.101) | 81 |
| Example 23 | 139 | 4.89 | 6.82 | (0.134, 0.100) | 81 |
| Example 24 | 150 | 4.87 | 6.90 | (0.133, 0.100) | 81 |
| Example 25 | 155 | 4.83 | 6.86 | (0.134, 0.101) | 87 |
| Comparativ e Example 1 | NPB | 5.52 | 6.05 | (0.134, 0.100) | 62 |
| Comparativ e Example 2 | Comparative compound A | 5.08 | 6.23 | (0.133, 0.100) | 61 |
| Comparativ e Example 3 | Comparative compound B | 5.11 | 6.24 | (0.133, 0.101) | 64 |
| Comparativ e Example 4 | Comparative compound C | 5.14 | 6.24 | (0.134, 0.100) | 63 |
| Comparativ e Example 5 | Comparative compound D | 5.09 | 6.12 | (0.133, 0.100) | 63 |
| Comparativ e Example 6 | Comparative compound E | 5.12 | 6.10 | (0.134, 0.101) | 60 |
| Comparativ e Example 7 | Comparative compound F | 5.13 | 6.14 | (0.133, 0.101) | 63 |
| Comparativ e Example 8 | Comparative compound G | 5.31 | 6.25 | (0.133, 0.100) | 66 |

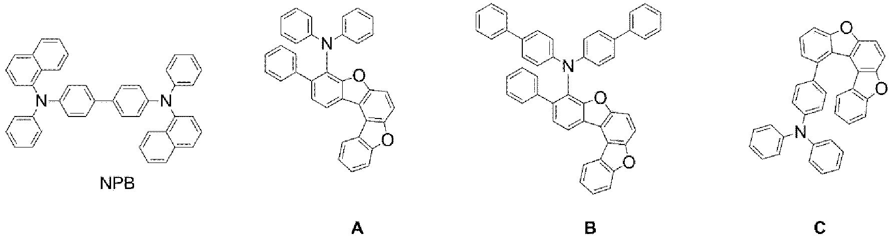

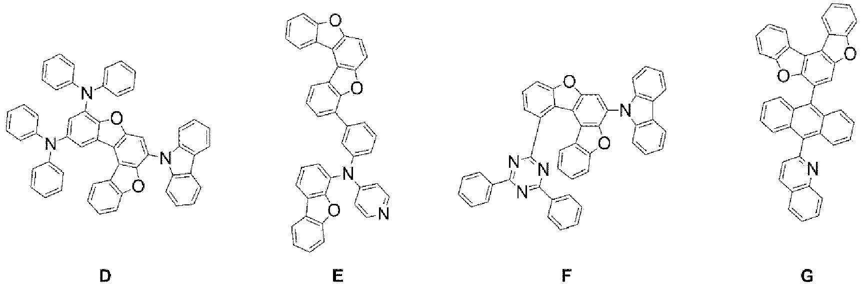

Referring to Table 6 above, the blue organic light-emitting device having a hole transport layer into which the heterocyclic compound of the present disclosure is introduced exhibited a lower operating voltage and higher luminous efficiency than the organic light-emitting devices of the comparative examples, and showed an improved lifetime.

The organic light-emitting devices of the examples exhibited T₉₅ lifetime values of 80 or more and an operating voltage of 4.90 V or less, thereby demonstrating enhanced operating efficiency. In contrast, the organic light-emitting devices of the comparative examples exhibited T₉₅ lifetime values of 66 or less and an operating voltage of 5.08 V or more, thereby showing degraded efficiency compared to the organic light-emitting devices of the examples.

The compounds used in the hole transport layers of the examples include an arylamine group bonded to the first ring of the 5-membered fused ring structure and an aryl group bonded to the third ring, which are not present in the compounds used in Comparative Examples 1 to 4. Accordingly, this may result from the higher intermolecular carrier mobility due to stronger intermolecular interactions compared to those of the compounds used in Comparative Examples 1 to 4.

In addition, the compounds used in the hole transport layers of the examples may exhibit improved carrier balance compared to those used in Comparative Examples 5 to 7. The compounds used in Comparative Examples 5 to 7 exhibit excessively strong electron transport (ET) properties, making them less suitable for use in hole transport layers, and the capacitance characteristics caused by excess electrons may not be appropriately implemented. As a result, they may exhibit inferior characteristics compared to the compounds used in the hole transport layers of the examples.

The compound used in Comparative Example 8 may have an energy level less suitable for use in the hole transport layer compared to the compounds used in the hole transport layers of the examples.

### Fabrication of Organic light-emitting device 2

Organic light-emitting device 2 was fabricated in the same manner as in the fabrication of Organic light-emitting device 1, except that the hole transport layer was formed as described below.

N,N'-bis(α-naphthyl)-N,N'-diphenyl-4,4'-diamine (NPB) was placed in another crucible in the vacuum deposition equipment, and a current was applied to the crucible to evaporate the NPB to form a hole transport layer having a thickness of 250 Å. Then, an electron blocking layer having a thickness of 50 Å, which includes a compound shown in Table 7, was formed on the hole transport layer.

### Experimental Example 2

The T₉₅, operating voltage, luminous efficiency, color coordinates (CIE), and lifetime of the organic light-emitting device were measured in the same manner as in Experimental Example 1, and the results are shown in Table 7. In addition, the LUMO levels and T1 levels of some compounds are shown in Table 8.

**[TABLE 7]**

| | Compound No. | Operating voltage (V) | Luminous efficiency (cd/A) | CIE (x, y) | Lifetime (T₉₅) |
|---|---|---|---|---|---|
| Example 26 | 003 | 4.84 | 6.83 | (0.134, 0.101) | 86 |
| Example 27 | 008 | 4.89 | 6.84 | (0.133, 0.100) | 91 |
| Example 28 | 017 | 4.84 | 6.81 | (0.133, 0.101) | 84 |
| Example 29 | 023 | 4.71 | 6.80 | (0.134, 0.100) | 86 |
| Example 30 | 026 | 4.86 | 6.88 | (0.134, 0.101) | 91 |
| Example 31 | 038 | 4.81 | 6.86 | (0.133, 0.100) | 88 |
| Example 32 | 048 | 4.84 | 6.85 | (0.133, 0.100) | 87 |
| Example 33 | 059 | 4.71 | 6.86 | (0.134, 0.100) | 86 |
| Example 34 | 064 | 4.71 | 6.90 | (0.134, 0.100) | 85 |
| Example 35 | 073 | 4.86 | 6.81 | (0.134, 0.101) | 84 |
| Example 36 | 078 | 4.81 | 6.88 | (0.133, 0.100) | 88 |
| Example 37 | 086 | 4.84 | 6.86 | (0.134, 0.100) | 85 |
| Example 38 | 093 | 4.71 | 6.80 | (0.133, 0.100) | 90 |
| Example 39 | 099 | 4.86 | 6.88 | (0.133, 0.100) | 90 |
| Example 40 | 113 | 4.79 | 6.81 | (0.134, 0.100) | 84 |
| Example 41 | 120 | 4.81 | 6.80 | (0.134, 0.100) | 86 |
| Example 42 | 127 | 4.84 | 6.88 | (0.133, 0.100) | 83 |
| Example 43 | 129 | 4.71 | 6.80 | (0.133, 0.100) | 88 |
| Example 44 | 133 | 4.86 | 6.88 | (0.134, 0.100) | 90 |
| Example 45 | 136 | 4.84 | 6.86 | (0.134, 0.100) | 81 |
| Example 46 | 137 | 4.71 | 6.81 | (0.133, 0.100) | 85 |
| Example 47 | 138 | 4.86 | 6.90 | (0.133, 0.100) | 86 |
| Example 48 | 140 | 4.84 | 6.88 | (0.133, 0.100) | 85 |
| Example 49 | 144 | 4.71 | 6.80 | (0.134, 0.100) | 83 |
| Example 50 | 149 | 4.86 | 6.88 | (0.134, 0.101) | 86 |
| Example 51 | 154 | 4.79 | 6.86 | (0.134, 0.100) | 88 |
| Comparative Example 9 | NPB | 5.48 | 6.07 | (0.134, 0.100) | 53 |
| Comparative Example 10 | Comparative compound H | 5.22 | 6.23 | (0.134, 0.100) | 53 |
| Comparative Example 11 | Comparative compound I | 5.27 | 6.16 | (0.133, 0.100) | 56 |
| Comparative Example 12 | Comparative compound J | 5.29 | 6.33 | (0.133, 0.100) | 60 |
| Comparative Example 13 | Comparative compound K | 5.21 | 6.24 | (0.133, 0.100) | 55 |
| Comparative Example 14 | Comparative compound L | 5.24 | 6.22 | (0.133, 0.101) | 51 |
| Comparative Example 15 | Comparative compound M | 5.22 | 6.31 | (0.134, 0.100) | 66 |
| Comparative Example 16 | Comparative compound N | 5.30 | 6.19 | (0.134, 0.101) | 63 |

**[TABLE 8]**

| | Compound No. | LUMO level | T1 level |
|---|---|---|---|
| Example 27 | 008 | -1.86 | 2.55 |
| Example 28 | 017 | -1.85 | 2.64 |
| Example 30 | 026 | -1.81 | 2.62 |
| Example 32 | 048 | -1.87 | 2.60 |
| Example 36 | 078 | -1.70 | 2.55 |
| Comparative Example 9 | NPB | -1.56 | 2.45 |
| Comparative Example 13 | Comparative compound K | -1.82 | 2.46 |
| Comparative Example 14 | Comparative compound L | -1.76 | 2.34 |
| Comparative Example 15 | Comparative compound M | -2.18 | 1.72 |

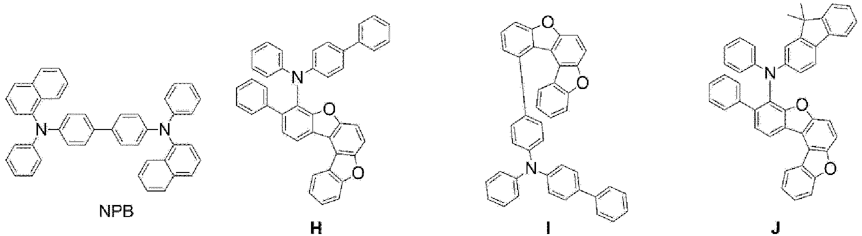

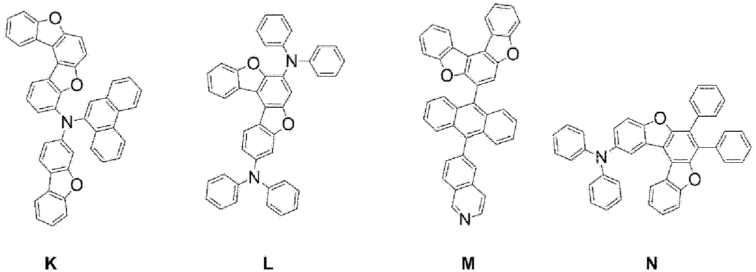

Referring to Table 7, the blue organic light-emitting device having an electron blocking layer into which the heterocyclic compound of the present disclosure is introduced exhibited a lower operating voltage, higher luminous efficiency, and improved lifetime compared to the organic light-emitting devices of the comparative examples.

The organic light-emitting devices of the examples exhibited T₉₅ lifetime values of 81 or more and an operating voltage of 4.89 V or less, thereby exhibiting improved operating efficiency. In contrast, the organic light-emitting devices of the comparative examples exhibited T₉₅ lifetime values of 66 or less and an operating voltage of 5.21 V or more, thereby showing degraded efficiency compared to the organic light-emitting devices of the examples.

Unlike the compounds used in Comparative Examples 9 to 13, the compounds used in the electron blocking layers of the examples include an arylamine group bonded to the first ring of the 5-membered fused ring and an aryl group bonded to the third ring.

Referring to Table 8 above, the compounds used in the electron blocking layers of the examples generally exhibited higher T1 levels than those used in the electron blocking layers of the comparative examples. Accordingly, the triplet energy generated from the light-emitting layer can be prevented from transferring to the electron blocking layer, thereby significantly improving the operating efficiency and lifetime of the organic light-emitting device. In addition, the compounds used in the electron blocking layers of the examples have LUMO levels suitable for forming the electron blocking layer, and can effectively block electron transport while efficiently transferring holes from the hole transport layer to the light-emitting layer.

This may be attributed to the higher intermolecular carrier mobility resulting from stronger intermolecular interactions of the compounds of the examples, compared to those used in Comparative Examples 9 to 13.

Furthermore, the compounds used in the electron blocking layers of the examples may exhibit improved carrier balance compared to the compound used in Comparative Example 14. The compound of Comparative Example 14 has an excessively strong electron transport (ET) tendency for use in the hole transport layer, and the resulting excess electron capacitance may not be properly implemented. As a result, its characteristics may be inferior to those of the compounds used in the hole transport layers of the examples.

The compound used in Comparative Example 15 may have an energy level that is less suitable for application in the hole transport layer than the compounds used in the hole transport layer of the examples.

The compound used in Comparative Example 16 has a structure in which two aryl groups are bonded to the third ring of the 5-membered fused ring. This may hinder intermolecular interactions and degrade the performance compared to the compounds used in the electron blocking layers of the examples.

Based on the above results, it was confirmed that the heterocyclic compounds of the present disclosure can improve the operating performance, efficiency, and lifetime characteristics of organic light-emitting devices.

The contents described above are merely examples of applying the principles of the present disclosure, and other configurations may be further included without departing from the scope of the present disclosure.

### [Description of Reference Numerals]

100: Substrate
200: Cathode
300: Organic layer
301: Hole injection layer
302: Hole transport layer
303: Light-emitting layer
304: Electron transport layer
305: Electron injection layer
400: Anode

## Claims

1. A heterocyclic compound represented by Formula 1 below: 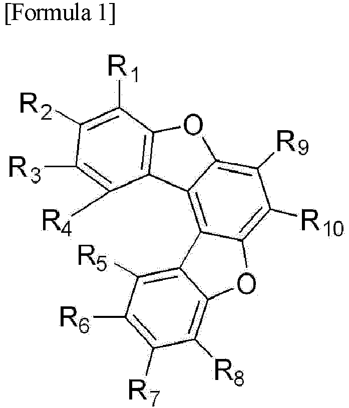 in Formula 1, one of R₁ to R₈ is a substituent represented by Formula 2 below, and
the others are each independently selected from the group consisting of hydrogen; deuterium; halogen; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -SiRR'R"; and -P(=O)RR',
R, R' and R" are each independently hydrogen; deuterium; -CN; a substituted or unsubstituted alkyl group having 1 to 60 carbon atoms; a substituted or unsubstituted cycloalkyl group having 3 to 60 carbon atoms; a substituted or unsubstituted aryl group having 6 to 60 carbon atoms; or a substituted or unsubstituted heteroaryl group having 2 to 60 carbon atoms, 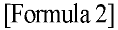 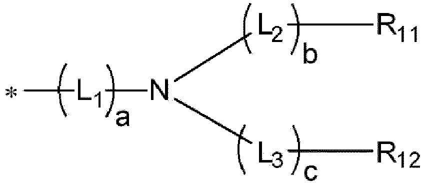
in Formula 2, R₁₁ and R₁₂ are each independently a substituted or unsubstituted aryl group having 6 to 60 carbon atoms, or a substituted or unsubstituted heteroaryl group having 2 to 60 carbon atoms,
L₁, L₂ and L₃ are each independently a direct linkage, a substituted or unsubstituted arylene group having 6 to 60 carbon atoms, or a substituted or unsubstituted heteroarylene group having 2 to 60 carbon atoms,
a, b and c are each independently 0 or an integer from 1 to 5,
when a is an integer from 2 to 5, a plurality of L₁ are the same or different,
when b is an integer from 2 to 5, a plurality of L₂ are the same or different,
when c is an integer from 2 to 5, a plurality of L₃ are the same or different,
one of R₉ and R₁₀ is -(L₄)n-R₁₃, and the other is hydrogen or deuterium,
L₄ is a direct linkage, a substituted or unsubstituted arylene group having 6 to 60 carbon atoms, or a substituted or unsubstituted heteroarylene group having 2 to 60 carbon atoms,
n is an integer from 1 to 5, and when n is an integer from 2 to 5, a plurality of L₄ are the same or different,
R₁₃ is a substituted or unsubstituted aryl group having 6 to 60 carbon atoms, or a substituted or unsubstituted heteroaryl group having 2 to 60 carbon atoms, and
* indicates a bonding site with an adjacent atom.

2. The heterocyclic compound according to claim 1, wherein in Formula 2 above,
L₁, L₂ and L₃ are each independently a direct linkage, or a substituted or unsubstituted arylene group having 6 to 10 carbon atoms, and
a, b and c are each independently 0, 1 or 2.

3. The heterocyclic compound according to claim 1, wherein the heterocyclic compound is represented by Formula 1-1 below: 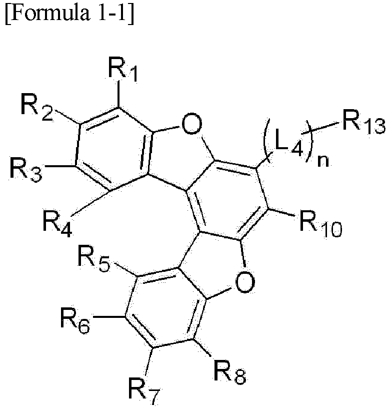 in Formula 1-1,
one of R₁ to R₄ is a substituent represented by Formula 2, and the others are each independently hydrogen or deuterium,
R₅ to R₈ and R₁₀ are each independently hydrogen or deuterium,
L₄ is a direct linkage, a substituted or unsubstituted arylene group having 6 to 60 carbon atoms, or a substituted or unsubstituted heteroarylene group having 2 to 60 carbon atoms,
n is an integer from 1 to 5, and when n is an integer from 2 to 5, a plurality of L₄ are the same or different, and
R₁₃ is a substituted or unsubstituted aryl group having 6 to 60 carbon atoms, or a substituted or unsubstituted heteroaryl group having 2 to 60 carbon atoms.

4. The heterocyclic compound according to claim 1, wherein in Formula 1 above,
one of R₉ and R₁₀ is -(L₄)n-R₁₃, and the other is hydrogen or deuterium,
L₄ is a direct linkage, or a substituted or unsubstituted arylene group having 6 to 20 carbon atoms,
n is an integer from 1 to 5, and when n is an integer from 2 to 5, a plurality of L₄ are the same or different, and
R₁₃ is a substituted or unsubstituted aryl group having 6 to 40 carbon atoms, or a substituted or unsubstituted heteroaryl group having 2 to 40 carbon atoms.

5. The heterocyclic compound according to claim 1, wherein Formula 2 above is represented by any one of Formula 2-1 and Formula 2-5 below: 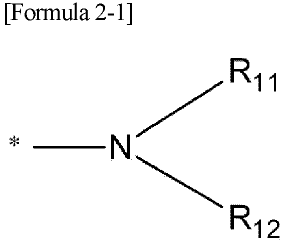 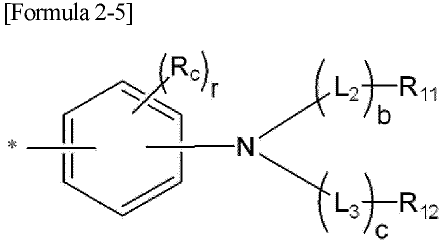
in Formula 2-1 and 2-5 above, R₁₁, R₁₂, L₂, L₃, b and c are the same as defined in Formula 2, R_{c} is hydrogen or deuterium, respectively, and r is 0 or an integer from 1 to 4, and
when r is an integer of 2 or more, a plurality of R_{c} are the same or different.

6. The heterocyclic compound according to claim 1, wherein the deuterium content of the heterocyclic compound is 0, or greater than 0 and less than or equal to 100%.

7. The heterocyclic compound according to claim 1, wherein the heterocyclic compound is represented by any one of the chemical formulas selected from the following:

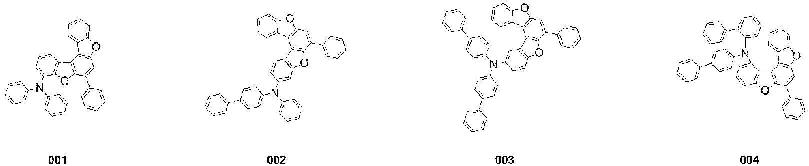

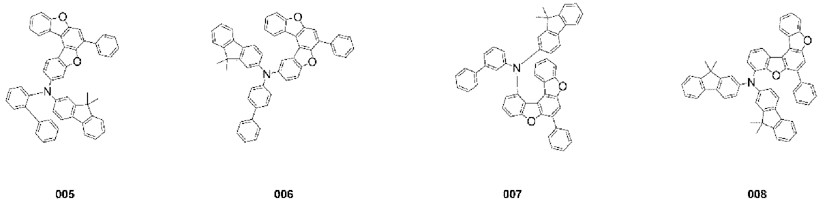

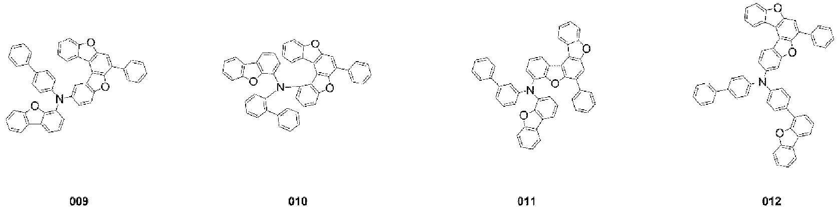

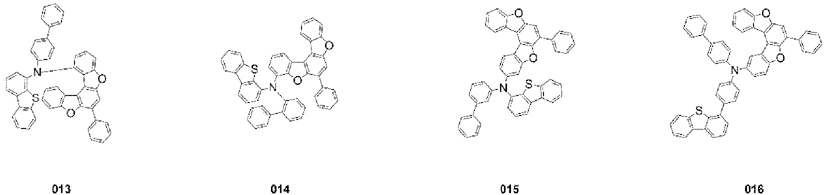

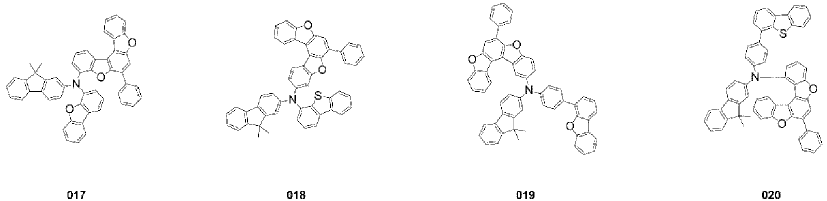

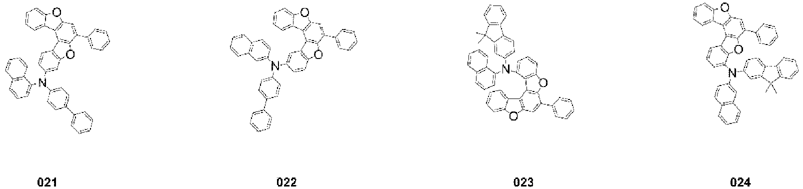

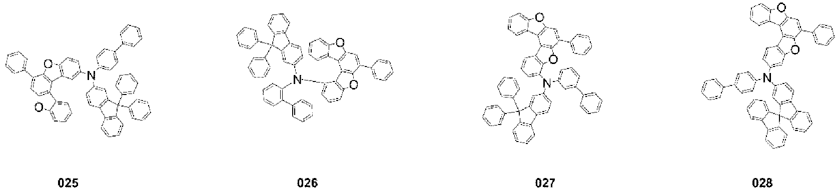

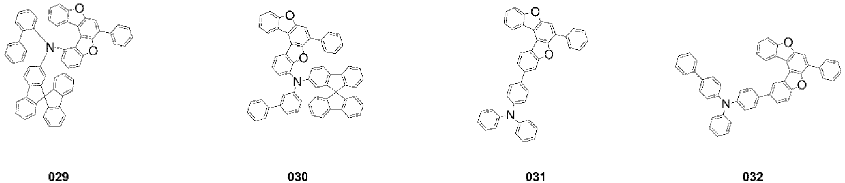

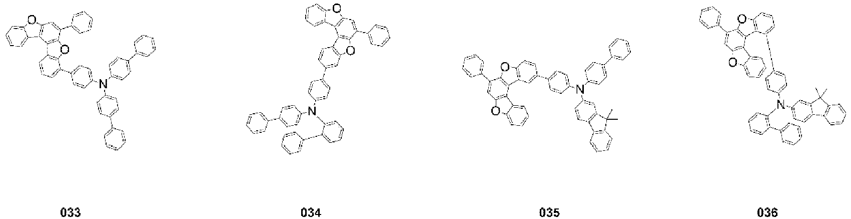

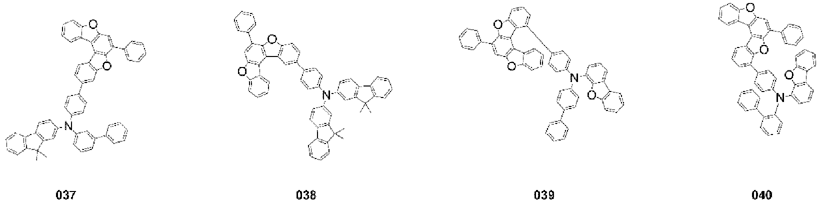

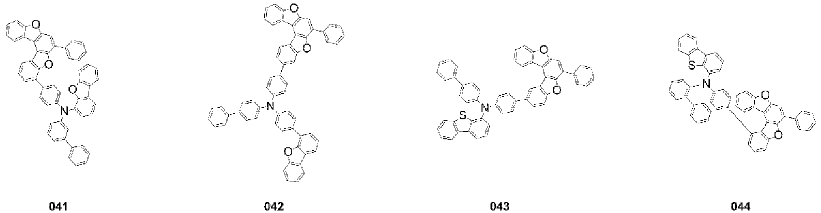

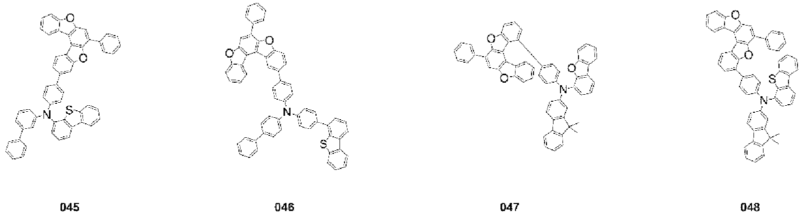

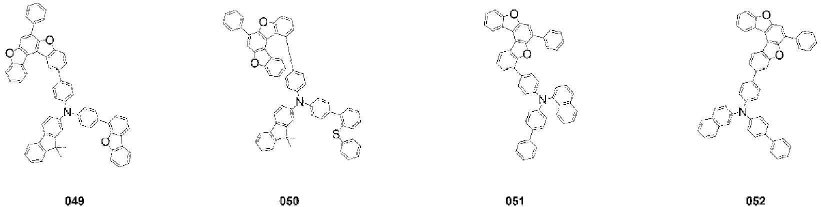

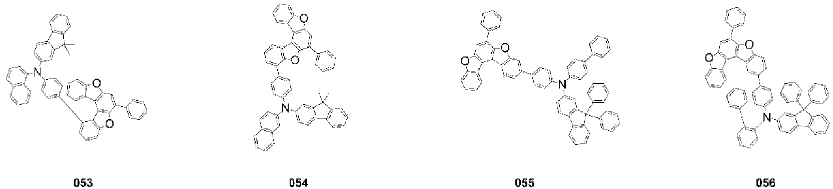

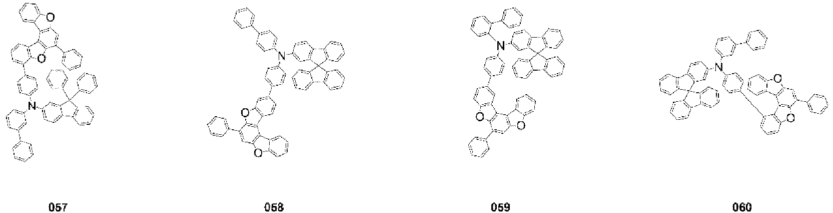

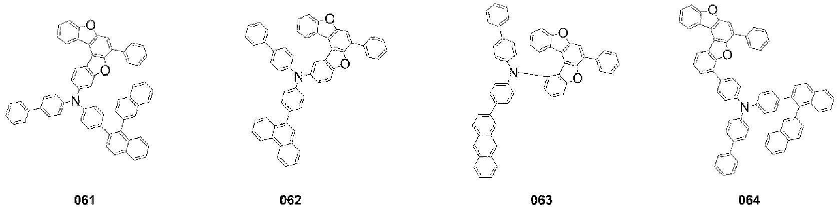

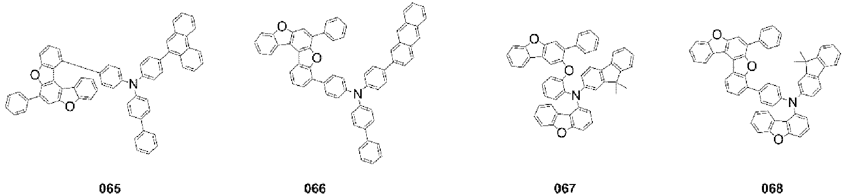

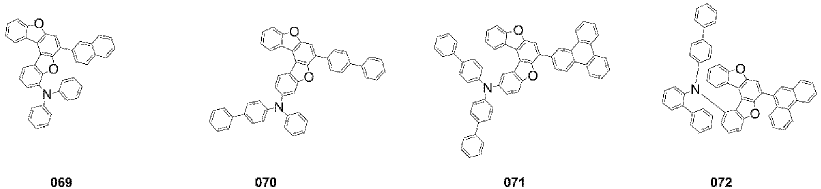

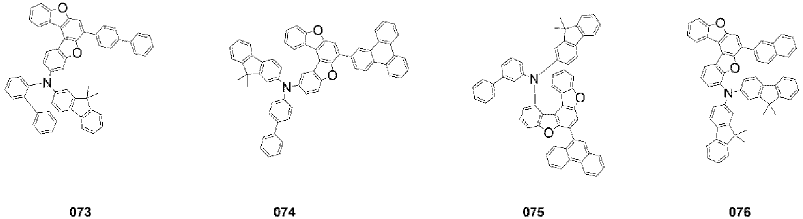

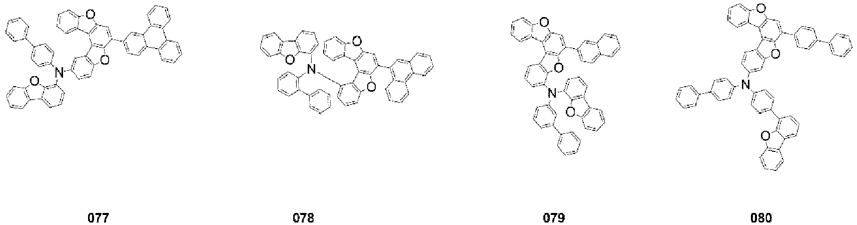

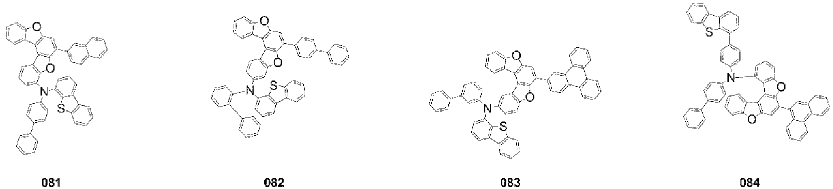

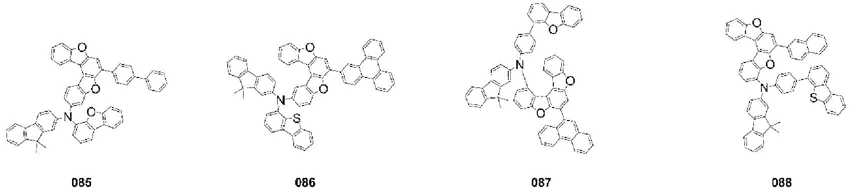

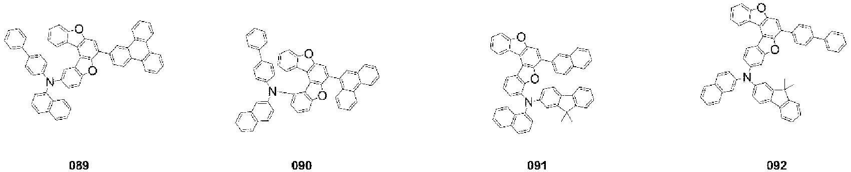

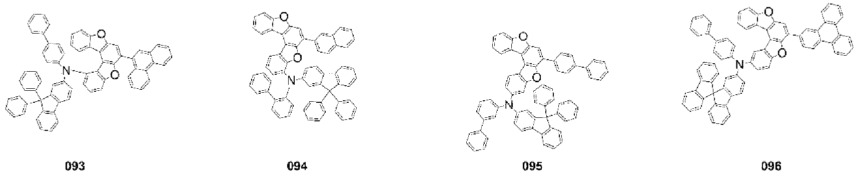

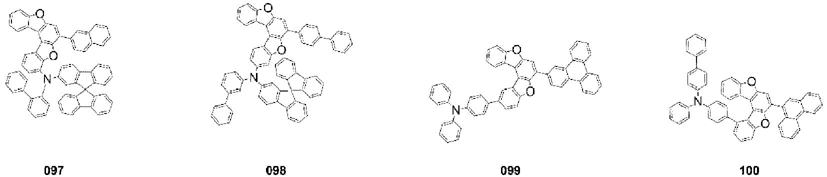

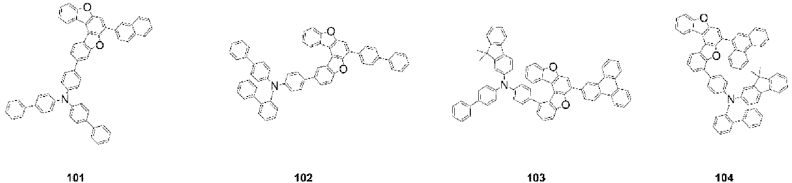

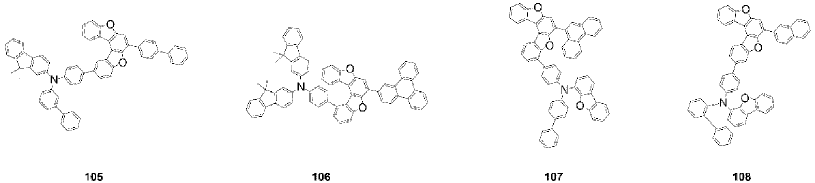

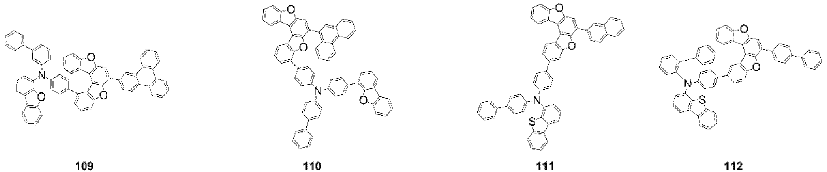

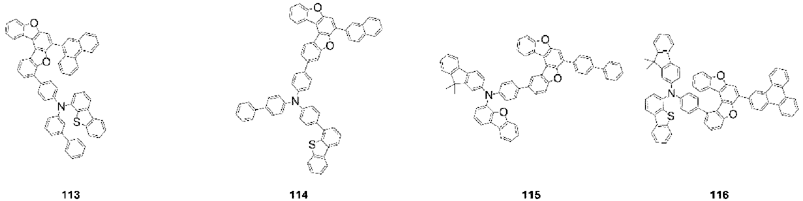

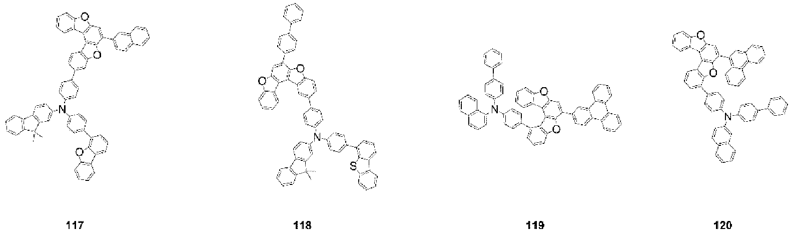

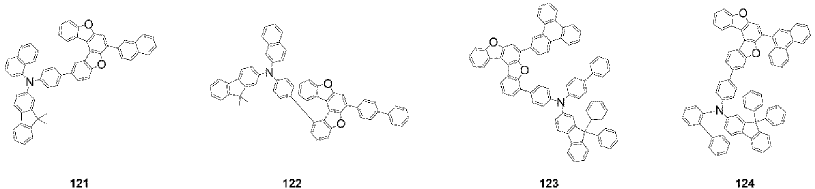

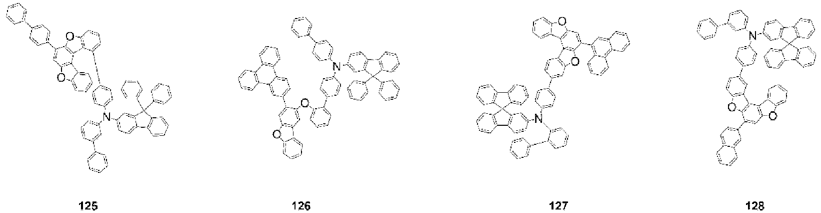

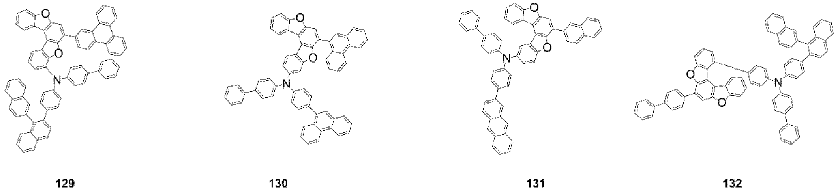

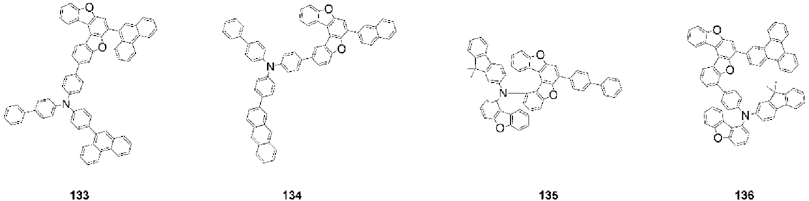

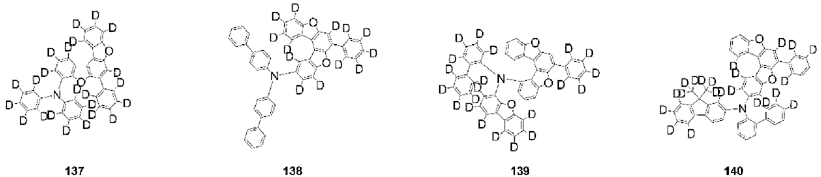

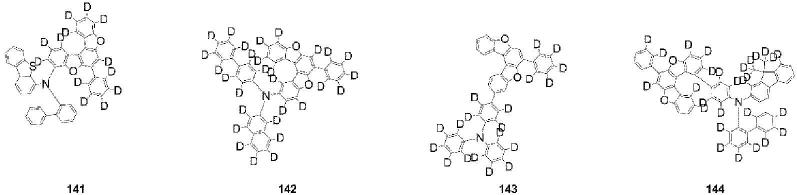

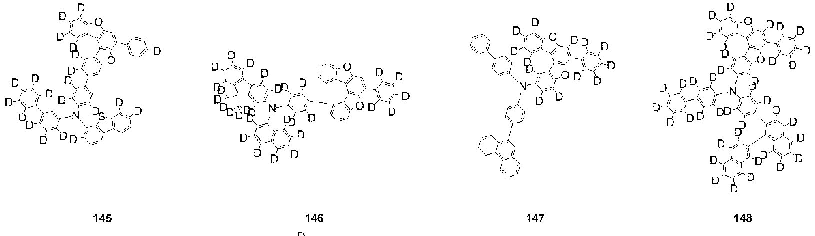

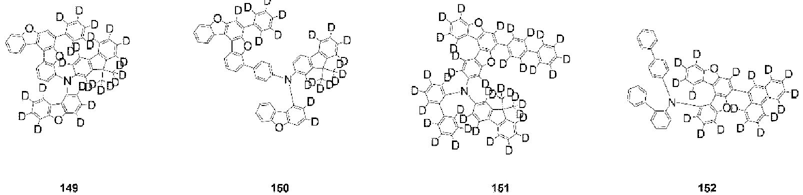

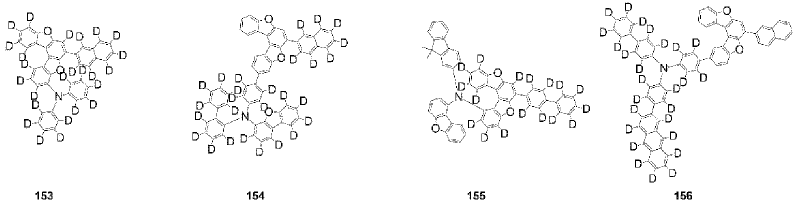

8. An organic light-emitting device comprising:
a first electrode;
a second electrode disposed on the first electrode; and
one or more organic layers interposed between the first electrode and the second electrode,
wherein one or more of the organic layers comprise the heterocyclic compound according to claim 1.

9. The organic light-emitting device according to claim 8, wherein the organic layer comprises: a light-emitting layer; a hole transporting layer disposed between the first electrode and the light-emitting layer; and an electron transporting layer disposed between the light-emitting layer and the second electrode.

10. The organic light-emitting device according to claim 9, wherein the hole transporting layer comprises a hole injection layer, a hole transport layer, an auxiliary hole transport layer, and an electron blocking layer, and at least one of the hole transport layer, the auxiliary hole transport layer, and the electron blocking layer comprises the heterocyclic compound.

11. The organic light-emitting device according to claim 9, wherein the electron transporting layer further comprises at least one layer selected from the group consisting of an electron injection layer, an electron transport layer, and an electron blocking layer.

12. The organic light-emitting device according to claim 8, wherein the organic layer further comprises one or more layers selected from the group consisting of a light-emitting layer, a hole injection layer, a hole transport layer, an auxiliary hole transport layer, an electron injection layer, an electron transport layer, an electron blocking layer, and a hole blocking layer.
